(19) Europäisches Patentamt

European Patent Office

Office européen des brevets

(11) **EP 1 090 643 A1**

(12) **EUROPEAN PATENT APPLICATION**
published in accordance with Art. 158(3) EPC

(43) Date of publication:
**11.04.2001 Bulletin 2001/15**

(21) Application number: **99957623.4**

(22) Date of filing: **25.06.1999**

(51) Int. Cl.[7]: **A61K 45/00**, A61K 39/395
// (C07K16/18, 16:46,
C12N15:62, 5:16, C12P21:08)

(86) International application number:
**PCT/JP99/03433**

(87) International publication number:
**WO 00/00219 (06.01.2000 Gazette 2000/01)**

(84) Designated Contracting States:
**AT BE CH CY DE DK ES FI FR GB GR IE IT LI LU
MC NL PT SE**

(30) Priority: **26.06.1998 JP 18014398**

(71) Applicant:
**CHUGAI SEIYAKU KABUSHIKI KAISHA
Tokyo, 115-8543 (JP)**

(72) Inventors:
• **SATO, Koh
Gotenba-shi Shizuoka 412-8543 (JP)**
• **TSUNENARI, Toshiaki
Gotenba-shi Shizuoka 412-8543 (JP)**

(74) Representative:
**Maschio, Antonio et al
D Young & Co,
21 New Fetter Lane
London EC4A 1DA (GB)**

(54) **REMEDIES FOR HYPERCALCEMIC CRISIS**

(57)    The invention provides a therapeutic agent for hypercalcemic crisis comprising, as an active ingredient, a substance capable of inhibiting the binding between a parathyroid hormone related protein (PTHrP) and a receptor thereof.

## Description

TECHNICAL FIELD

The present invention relates to a therapeutic agent for hypercalcemic crisis associated with malignant tumor, which comprises a substance capable of inhibiting the binding of parathyroid hormone related protein (PTHrP) to a receptor thereof as an active ingredient.

BACKGROUND ART

Hypercalcemia associated with malignant tumor is a serious complication which occurs in 5 to 20% of the total number of patients with malignant tumor and can be fatal with certainty if untreated. Accordingly, hypercalcemia has been considered as a terminal symptom of malignant tumor. Control of hypercalcemia largely influences the therapeutic prognosis and QOL (Quality of Life) of the patient and, therefore, plays a clinically important role.

In general, hypercalcemia found in patients with malignant tumor is broadly divided into two types: humoral hypercalcemia of malignancy (HHM) which is caused by a tumor-producing humoral bone-resorbing factor; and local osteolytic hypercalcemia (LOH) which is caused by a topical action of tumor which metastasizes to or infiltrates into bones. In HHM, it has been considered that excretion of calcium is increased by acceleration of bone resorption or destruction, resulting in development of hypercalcemia in synergy with decreased renal excretory capacity of calcium (see Seiki Wada and Naokazu Nagata, Naika 69, 644-648).

Hypercalcemia is considered to be manifested when serum calcium level exceeds 12 mg/dL, and produce disgust at the sight or thought of food, nausea and vomiting in patients with malignant tumor nonspecifically in its early stage. When hypercalcemia becomes worsened, polyuria may occur due to the decreased fluid-concentrating ability caused by distal renal tubular disorder, dehydration may associates due to the insufficient intake of water resulted from nausea or vomiting, and calcification may occur in the kidney, skin, blood vessels, lung, heart and stomach. When hypercalcemia becomes more worsened, impaired consciousness such as general malise, rethargy and confusion occurs, which may eventually result in coma and cardiac arrest. (see Harrison's Text for Internal Medicine, 3427, Internal Medicine, 1081-1084, Asakura Shoten.)

As a humoral factor which causes HHM (one type of the malignancy-associated hypercalcemia), parathyroid hormone-related peptide (hereinafter, simply referred to as "PTHrP"), which is a parathyroid hormone(PTH)-like substance, was found by Moseley, J. M. et al (Proc. Natl. Acad. Sci. USA. (1987) 84, 5048-5052).

Thereafter, a gene encoding PTHrP was isolated (Suva, L. J. et al., Science (1987) 237, 893). The analysis of the gene revealed that there are three types of human PTHrP comprising 139, 141 and 173 amino acid residues, respectively, depending on the fashion of selective splicing of the gene and that various fragments produced by the limited cleavage of the entire PTHrP (1-139) are present in the blood (Baba, H., Clinical Calcium (1995) 5, 229-223). In PTHrP, it is assumed that 8 out of 13 amino acids of the N-terminal amino acids 1-13 are identical to those of PTH and a sequence comprising amino acids 14-34 has the three dimensional structure similar to that of PTH, and at the N-terminal region of PTHrP can bind to PTH/PTHrP receptors which are shared by PTH (Jueppner, H. et al., Science (1991) 254, 1024-1026; Abou-Samra, A-B, et al., Proc. Natl., Acad. Sci. USA (1992) 89, 2732-2736).

PTH/PTHrP receptors are present primarily in bones and the kidney (Chohei Sigeno, Clinical Calcium (1995) 5, 355-359), and it is known that a plurality of intracellular signal transduction systems can be activated through the binding of PTHrP to its receptors. One of the intracellular signal transduction systems is adenyl cyclase and the other is phospholipase C. Adenyl cyclase, when activated, causes the rise in intracellular cAMP concentration, thereby inducing the activation of protein kinase A. Phospholipase C cleaves phosphatidylinositol 4,5-bisphosphonate to produce inositol 1,4,5-triphosphonate and diacylglycerol. G protein involves in these signal transduction systems (Coleman, D. T. et al., Biochemical mechanisms of parathyroid hormone action. In: "The parathyroids" (Bilezikian, J. P. et al), Raven press, New York (1994) p.239). PTHrP can cause hypercalcemia, hypophosphatemia, reduced renal phosphorus-resorbing capacity, increased renal cAMP excretion and so on which are observed in HHM.

As mentioned above, PTHrP not only closely relates to hypercalcemia associated with malignant tumor, but also causes hypercalcemic crisis including impaired consciousness (e.g., general malise, rethargy and confusion) and coma resulted from rapid increase in blood calcium level. When hypercalcemic crisis associated with malignant tumor is developed, calcitonin, a steroid, a bisphosphonate, phosphate buffer, physiological saline, furosemide or the like is administered to improve hypercalcemia and general conditions. However, these drugs have such disadvantages that therapeutic effects may be depressed when successively administered, that severe adverse side effects may be produced, and that the development of pharmacological effects may be delayed. Accordingly, use of drugs having higher therapeutic effects and fewer side effects have been expected.

Particularly in patients with hypercalcemic crisis, since the rapid increase in blood calcium level may sometimes lead to death, it is needed to decrease the blood calcium level as soon as possible. However, up to the present, no drug is known which is satisfactory as a safe, prompt therapeutic agent for hypercalcemic crisis.

As a new approach to treating hypercalcemia associated with malignant tumor, it has been reported by Kukreja, S. C. et al that when a neutralizing antiserum against PTHrP was administered to an athymic mouse into which human lung cancer cells or human pharyngeal cancer cells had been transplanted to cause hypercalcemia, the blood calcium level and urinary cAMP level were decreased (J. Clin. Invest. (1988) 82, 1798-1802). It has also been reported by Kanji Sato et al. that when an antibody against PTHrP (1-34) was administered to a nude mouse into which PTHrP-producing human tumor had been transplanted, hypercalcemia was ameliorated and the surviving period of the mouse was greatly prolonged (J. bone & Mine. Res. (1993) 8, 849-860). Japanese Patent Application Laid-open No. 4-228089 discloses a mouse/human chimeric antibody against human PTHrP (1-34).

As mentioned above, humanized antibodies are expected to be useful for use in emergency treatment of hypercalcemic crisis associated with malignant tumor. However, humanized antibody against PTHrP is not yet known and the above documents do not suggest such humanized antibodies. Moreover, there is no method for producing humanized antibodies which is universally applicable to any antibody. Therefore, various modifications or contrivances are needed in the production of humanized antibodies having satisfactory ability to bind to and neutralizing a specific antigen (see, for example, Sato, K. et al., Cancer Res., 53, 851-856, 1993).

DISCLOSURE OF INVENTION

The object of the present invention is to provide a therapeutic agent for hypercalcemic crisis, which comprises, as an active ingredient, a substance capable of inhibiting the binding between parathyroid hormone related protein (PTHrP) and a receptor thereof.

The present inventors have been made extensive and intensive studies on development of such a therapeutic agent. As a result, the inventors have found that the use of a substance capable of inhibiting the binding of PTHrP to a receptor thereof can achieve the object. This finding led to the accomplishment of the invention.

That is, the present invention relates to a therapeutic agent for hypercalcemic crisis comprising, as an active ingredient, a substance capable of inhibiting the binding between PTHrP and a receptor thereof. The substance includes an antagonist against a PTHrP receptor and an anti-PTHrP antibody. The substance may be any fragment of an anti-PTHrP antibody and/or any modified form of the fragment. The antibody (including a monoclonal type) may be of a humanized or chimeric form (e.g.; as for a humanized antibody, humanized #23-57-137-1 antibody).

The present invention also relates to a therapeutic agent for hypercalcemic crisis associated with malignant tumor comprising, as an active ingredient, a substance capable of inhibiting the binding between PTHrP and a receptor thereof.

Hereinbelow, the invention will be described in detail.

The present invention relates to a therapeutic agent for hypercalcemic crisis comprising, as an active ingredient, a substance capable of inhibiting the binding between parathyroid hormone related protein (hereinafter, referred to as "PTHrP") and a receptor thereof.

As used herein, the term "a substance capable of inhibiting the binding between PTHrP and a receptor thereof" refers to any substance that can bind to PTHrP to inhibit the binding of the PTHrP to a PTHrP receptor or any substance that can bind to a PTHrP receptor to inhibit the binding of PTHrP to the PTHrP receptor. A specific example of the former substance is an anti-PTHrP antibody, and a specific example of the latter substance is an antagonist against a PTHrP receptor (which is also referred to as "a PTHrP antagonist").

The PTHrP antagonist includes a polypeptide and a low molecular weight substance. For example, substances that can bind to a PTHrP receptor in an antagonistic manner against PTHrP, such as polypeptides as described in Japanese Patent Application Laid-open No. 7-165790, Peptides (UNITED STATES), 1995, 16(6) 1031-1037, Biochemistry (UNITED STATES) Apr. 28 1992, 31(16) 4026-4033, and Japanese National Phase Laid-open No. 5-509098, are included in the PTHrP antagonist. These polypeptides can be chemically synthesized according to a conventional manner.

The PTHrP antagonists are capable of competing with a naturally occurring PTHrP peptide to bind to a PTHrP receptor but inhibiting the signal transduction which occurs after the binding to thereby prevent the rise in calcium level.

The polypeptides may have mutation therein (e.g., deletion, replacement, addition or insertion) of at least one amino acid residue as long as they can exhibit equivalent PTHrP antagonistic activities, which are also encompassed in the PTHrP antagonists of the present invention.

Preferably, the therapeutic agent for hypercalcemic crisis according to the present invention can decrease the calcium level rapidly after the administration of the agent at a dose of preferably 0.1 to 10,000 mg/body, more preferably 0.5 to 1,000 mg/body, still more preferably 1 to 100 mg/body per patient and prevent the re-rise in the calcium level for a prolonged time period or exert a significantly long duration of action. Specifically, an agent used as the therapeutic agent of the present invention is one which can decrease the corrected serum calcium level by 1 mg/dL or more or can normalize the corrected serum calcium level (the corrected serum calcium level: 10.4 mg/dL or lower) or decrease the corrected serum calcium level by 2 mg/dL or more, within 24 hours, preferably within 6 hours, more preferably within 4 hours after the administration of the agent. More preferably, the agent can maintain a state where the corrected serum calcium level does not re-rise or can maintain a state where the corrected serum calcium level is decreased by 2 mg/dL or more compared with that before the administration of the agent or normalized the corrected serum calcium level, over 24 hours, preferably 3 days, more preferably 5 days, still more preferably 7 days, most preferably 10 days after the administration of the agent.

The "corrected serum calcium level" as used herein can be calculated according to either of the following equations depending on the serum albumin level:

(1) Serum albumin level of lower than 4 g/dL:

Corrected serum calcium level (mg/dL) = Measured serum calcium level (mg/dL) + [4.0 - serum albumin level (g/dL)]

(2) Serum albumin level of 4 g/dL or higher:

Corrected serum calcium level (mg/dL) = Measured serum calcium level (mg/dL)

BEST MODE FOR CARRYING OUT THE INVENTION

Hereinbelow, the present invention will be described in more detail exemplary using an anti-PTHrP antibody as the "substance capable of inhibiting the binding between PTHrP and a PTHrP receptor."

The anti-PTHrP antibody includes, for example, known antibodies such as a humanized antibody, a human antibody (WO 96/33735) and a chimeric antibody (Japanese Patent Application Laid-open No. 4-228089), as well as the antibody disclosed in the present invention (#23-57-137-1 antibody). The antibody may be a polyclonal type, but is preferably of a monoclonal type.

1. Anti-PTHrP antibody

The anti-PTHrP antibody used in the present invention may be any one as long as it can exhibit a therapeutic effect on hypercalcemic crisis associated with malignant tumor, regardless of its source, type (monoclonal or polyclonal) and configuration.

The anti-PTHrP antibody used in the present invention can be produced by a known method in the form of a polyclonal or monoclonal antibody. Preferably, the anti-PTHrP antibody is a monoclonal antibody derived from a mammal. The monoclonal antibody from a mammal includes those produced from a hybridoma and those produced by a genetic engineering technique from a host which is transformed with a recombinant expression vector carrying a gene for the antibody. The antibody used in the present invention is one that can bind to PTHrP to inhibit the binding of the PTHrP to a PTH/PTHrP receptor, thus blocking the signal transduction of the PTHrP and consequently inhibiting the biological activity of PTHrP.

A specific example of the antibody is #23-57-137-1 antibody which can be produced from a hybridoma clone #23-57-137-1.

The hybridoma clone #23-57-137-1 has been designated "mouse-mouse hybridoma #23-57-137-1" and deposited under the terms of the Budapest Treaty on August 15, 1996 at the National Institute of Bioscience and Human-technology, Agency of Industrial Science and Technology, Japan (1-3, Higashi 1-chome, Tsukuba-shi, Ibaraki, Japan) under the accession No. FERM BP-5631.

2. Antibody-producing hybridoma

A monoclonal antibody-producing hybridoma can basically be produced by a known technique. That is, PTHrP is used as an antigen for immunization in accordance with a conventional immunization method. The resultant immunocytes are fused to known parent cells by a conventional cell fusion method, and monoclonal antibody-producing cells are screened from the fused cells by a conventional screening method.

More specifically, the monoclonal antibody-producing cell can be prepared as follows.

First, human PTHrP, which is used as an sensitizing antigen for producing the antibody, is prepared by expressing the PTHrP gene/amino acid sequence disclosed in Suva, L. J. et al., Science (1987) 237, 893. That is, a nucleotide sequence encoding the PTHrP is inserted into any known expression vector, and a suitable host cell is transformed with the expression vector. The PTHrP protein is then isolated and purified from the transformed host cell or from a culture supernatant of the transformed host cell by any known method.

Then, the purified PTHrP protein is used as a sensitizing antigen. Alternatively, a 34-amino acid peptide (SEQ ID NO: 75) of the N-terminal region of the PTHrP may be used as a sensitizing antigen, which can be chemically synthesized.

The mammal to be immunized with the sensitizing antigen is not particularly limited. However, the mammal is preferably selected taking into consideration of compatibility with the parent cell used for cell fusion. Generally, a rodent (e.g., mouse, rat, hamster) or rabbit or monkey may be used.

The immunization of the mammal with the sensitizing antigen can be performed in accordance with any known method, for example, by injecting the sensitizing antigen to a mammal intraperitoneally or subcutaneously. More specifically, the sensitizing antigen is diluted with and suspended to phosphate-buffered saline (PBS) or physiological saline properly, the resultant suspension is then mixed with an appropriate amount of an adjuvant (e.g., Freund's complete adjuvant) to give an emulsion. The emulsion is injected to a mammal several times at intervals of 4 to 21 days. In the immunization, the sensitizing antigen may be attached to a suitable carrier.

After the immunization, the serum antibody level is checked. When the serum antibody level is confirmed to reach the desired level, immunocytes are isolated from the mammal and then subjected to cell fusion. A preferable immunocyte is a spleen cell.

The parent cell used for the cell fusion (i.e., the counterpart of the cell fusion with the immunocyte) is a myeloma cell derived from a mammal. The myeloma cell is of any known cell line, and, for example, P3 (P3x63Ag8.653) (J. Immnol. (1979) 123, 1548-1550), P3x63Ag8U.1 (Current Topics in Microbiology and Immunology (1978) 81, 1-7), NS-1 (Kohler, G. and Milstein, C. Eur. J. Immunol. (1976) 6, 511-519), MPC-11 (Margulies, D. H. et al., Cell (1976) 8, 405-415), SP2/0 (Shulman, M. et al., Nature (1978) 276, 269-270), FO (de St. Groth, S. F. et al., J. Immunol. Methods (1980) 35, 1-21), S194 (Trowbridge, I. S., J. Exp. Med. (1978) 148, 313-323) or R210 (Galfre, G. et al., Nature (1979) 277, 131-133).

Cell fusion of the immunocyte to the myeloma cell is basically performed in accordance with a known method such as the method of Milstein et al. (Kohler, G. and Milstein, C., Methods Enzymol. (1981) 73, 3-46) may be preferably used.

More specifically, the cell fusion is performed, for example, in a conventional nutrient culture medium in the presence of a cell fusion promoter. The cell fusion promoter may be polyethylene glycol (PEG) or a Sendai virus (hemagglutinating virus of Japan; HVJ). If desired, for the purpose of improving the fusion efficiency, an additive such as dimethyl sulfoxide may also be incorporated.

The ratio between the immunocytes and the myeloma cells for the cell fusion may be any one. For example, the immunocytes are used in the amount 1-10 times larger than the myeloma cells. The culture medium used for the cell fusion is, for example, RPMI 1640 medium or MEM medium suitable for the growth of the myeloma cell line, or other medium conventionally used for the culture of such cells. If desired, a serum supplement, such as feral calf serum (FCS), may be added to the culture medium.

The cell fusion is performed by well mixing the immunocytes and the myeloma cells of given amounts in the culture medium, adding PEG solution (e.g., mean molecular weight: about 1000-6000) (which has been previously warmed to about 37°C) thereto usually to a concentration of 30-60% (w/v), and then mixing the resultant solution, thereby giving fusion cells (hybridomas). Subsequently, an appropriate culture medium is added to the culture solution, and centrifuged to remove the supernatant. This procedure is repeated several times to remove the cell fusion promoter or the like that are undesirable for the growth of the hybridomas.

The obtained hybridomas can be selected by cultivating in a conventional selective medium, such as hypoxanthine-aminopterin-thymidine (HAT) medium. The culture of the hybridomas in HAT medium is performed for the time of period enough to cause to death of the cells other than the desired hybridomas (i.e., cells that fail to fuse), usually for several days to several weeks. Subsequently, a conventional limiting dilution method is performed to screen and monoclone the hybridomas that are secreting the desired antibody.

Alternatively, a human antibody having a binding activity against the PTHrP may be prepared by sensitizing a human lymphocyte with PTHrP *in vitro,* and then subjecting the sensitized lymphocyte to cell fusion to a human-derived myeloma cell capable of infinite growth (Japanese Patent Publication No. 1-59878). Alternatively, a human antibody against PTHrP may be prepared by injecting PTHrP as an antigen to a transgenic animal that has the entire repertories of the human antibody genes to give an anti-PTHrP antibody-producing cell, and immortalizing the cells, thus the human antibody can be produced from the immortalized cell (International Publication Nos. WO 94/25585, WO 93/12227, WO 92/03918 and WO 94/02602).

The monoclonal antibody-producing hybridoma prepared as above can be subcultured in a conventional culture medium and stored under liquid nitrogen for a long time of period.

For the production of a monoclonal antibody from the hybridoma, there may be employed a method involving cultivating the hybridoma in accordance with a conventional method and collecting the monoclonal antibody from the culture supernatant, or a method involving injecting the hybridoma to a mammal compatible with the hybridoma to grow the hybridoma in the mammal body and collecting the hybridoma from the ascites of the mammal. The former method is suitable for producing the antibody with high purity, while the latter method is suitable for producing the antibody in a large amount.

3. Recombinant antibody

In the present invention, a recombinant-type monoclonal antibody may also be used, which can be produced by cloning an antibody gene from the hybridoma, integrating the antibody gene into a suitable vector, introducing the vector into a host, and producing the antibody from the host according to a conventional genetic recombination technique (see, for example, Vandamme, A. M. et al., Eur. J. Biochem. (1990) 192, 767-775)

More specifically, mRNA encoding variable (V) region of an anti-PTHrP antibody is isolated from the anti-PTHrP antibody-producing hybridoma. The isolation of the mRNA may be performed by preparing a total RNA by any known method, such as guanidium ultracentrifugation method (Chirgwin, J. M. et al., Biochemistry (1979) 18, 5294-5299) and AGPC method (Chomczynski, P. et al., Anal. Biochem. (1987) 162, 156-159), and then producing the desired mRNA from the total RNA using mRNA Purification Kit (Pharmacia) or the like. Alternatively, the mRNA may also be prepared directly using QuickPrep mRNA Purification Kit (Pharmacia).

Next, cDNA for the antibody V-region is synthesized from the mRNA with a reverse transcriptase. The synthesis of the cDNA is performed using AMV Reverse Transcriptase First-strand cDNA Synthesis Kit (Seikagaku Corporation) or the like. The cDNA may also be synthesized or amplified by 5'-RACE method (Frohman, M.A. et al., Proc. Natl. Acad. Sci. USA (1988) 85, 8998-9002; Belyavsky, A. et al., Nucleic Acids Res. (1989) 17, 2919-2932) using 5'-Ampli FINDER RACE Kit (Clonetech) in combination with a PCR method, or the like.

A DNA fragment of interest is isolated and purified from the resultant PCR product and then ligated to a vector DNA to give a recombinant vector. The recombinant vector is introduced into a host such as *E. coli,* and a colony containing a desired recombinant vector is selected. The nucleotide sequence of the DNA of interest in the recombinant vector is confirmed by, for example, dideoxynucleotide chain termination method.

Once DNA encoding the anti-PTHrP antibody V-region is obtained, the DNA is integrated into an expression vector containing DNA encoding the antibody constant (C) region.

For the production of the anti-PTHrP antibody used in the present invention, the antibody gene is integrated into an expression vector so that the antibody gene can be expressed under the control of expression control regions (e.g., enhancer, promoter). A host cell is transformed with the expression vector to express the antibody.

In the expression of the antibody gene, DNA encoding heavy (H) chain and DNA encoding light (L) chain of the antibody may be integrated into separate expression vectors, and then a host cell is co-transformed with the resultant recombinant expression vectors. Alternatively, both DNA encoding H-chain and DNA encoding L-chain of the antibody may be integrated together into a single expression vector, and then a host cell is transformed with the resultant recombinant expression vector (WO 94/11523).

In the production of the recombinant antibody, besides the above-mentioned host cells, a transgenic animal may also be used as a host. For example, the antibody gene is inserted into a predetermined site of a gene encoding a protein which is inherently produced in the milk of an animal (e.g., goat β-casein), thereby producing a fusion gene. A DNA fragment containing the antibody gene-introduced fusion gene is injected into an embryo of a goat, and the embryo is then introduced into a female goat. The female goat having the embryo therein can bear a transgenic goat. The antibody of interest is secreted in the milk from the transgenic goat or a progeny thereof. For the purpose of increasing the amount of the antibody-containing milk, an appropriate hormone may be administered to the transgenic goat (Ebert, K.M. et al., Bio/Technology (1994) 12, 699-702).

4. Modified antibody

In the present invention, for the purpose of reducing the heterogenisity against a human body or the like, an artificially modified recombinant antibody may be used, such as a chimeric antibody and a humanized antibody. These modified antibodies can be prepared by any known method.

A chimeric antibody usable in the present invention can be prepared by ligating the DNA encoding the antibody V-region prepared as mentioned above to DNA encoding a human antibody C-region, integrating the ligation product into an expression vector, and introducing the resultant recombinant expression vector into a host, thereby producing the chimeric antibody.

A humanized antibody is also referred to as "reshaped human antibody", in which the complementarity determining regions (CDRs) of an antibody of a non-human mammal (e.g., a mouse) are grafted to those of a human antibody. The general genetic recombination procedure for producing such humanized antibody is also known (EP 125023; WO 96/02576).

Specifically, a DNA sequence in which mouse antibody CDRs are ligated through framework regions (FRs) is designed, and synthesized by a PCR method using several oligonucleotides as primers which were designed to have regions overlapping to the terminal regions of the CDRs and the FRs. The resultant DNA is ligated to DNA encoding the human antibody C-region, and the ligation product is integrated into an expression vector. The resultant recombinant expression vector is introduced into a host, thereby producing the humanized antibody (EP 239044, WO 96/02576).

The FRs ligated through the CDRs are selected so that the CDRs can form a satisfactory antigen binding site. If necessary, an amino acid(s) in the FRs of the antibody V-region may be replaced so that the CDRs of the reshaped human antibody can form an appropriate antigen binding site (Sato, K. et al., Cancer Res. (1993) 53, 851-856).

The C-region of the chimeric or humanized antibody may be any human antibody C-region; such as Cγ1, Cγ2, Cγ3 or Cγ4 for the H-chain, and Cκ or Cλ for the L-chain. The human antibody C-region may be modified for the purpose of improving the stability of the antibody or ensuring the stable production of the antibody.

The chimeric antibody is composed of V-regions derived from a non-human mammal antibody and C-regions derived from a human antibody. The humanized antibody is composed of CDRs derived from a non-human mammal antibody and FRs and C-regions derived from a human antibody. The humanized antibody is especially useful as an active ingredient for the therapeutic agent of the present invention, because the antigenicity of the antibody against a human body is reduced.

A specific example of the humanized antibody used in the present invention is humanized #23-57-137-1 antibody; in which the CDRs are derived from mouse-derived #23-57-137-1 antibody; and the L-chain is composed of the CDRs ligated through three FRs (FR1, FR2 and FR3) derived from human antibody HSU 03868 (GEN-BANK, Deftos, M. et al., Scand. J. Immunol., 39, 95-103, 1994) and a FR (FR4) derived from human antibody S25755 (NBRF-PDB); and the H-chain is composed of the CDRs ligated through FRs derived from human antibody S31679 (NBRF-PDB, Cuisinier, A. M. et al., Eur. J. Immunol. 23, 110-118, 1993) in which a portion of the amino acid residues in the FRs is replaced so that the reshaped humanized antibody can exhibit an antigen-binding activity.

The *E. coli* strains containing the plasmids having DNA encoding the H-chain and the L-chain of the humanized #23-57-137-1 antibody, respectively, are designated *Escherichia coli* JM109 (hMBC1HcDNA/pUC19) (for H-chain) and *Escherichia coli* JM109 (hMBC1Lqλ/pUC19) (for L-chain), respectively. These strains have been deposited under the terms of the Budapest Treaty on August 15, 1996 at the National Institute of Bioscience and Human-technology, Agency of Industrial Science and Technology, Japan (1-3, Higashi 1-chome, Tsukuba-shi, Ibaraki, Japan), under the accession No. FERM BP-5629 for *Escherichia coli* JM109 (hMBC1HcDNA/pUC19), and under the accession No. FERM BP-5630 for *Escherichia coli* JM109 (hMBC1Lqλ/pUC19).

5. Antibody variants

The antibody used in the present invention may be any fragment thereof or a modified product of the fragment, as long as it can bind to PTHrP and inhibit the activity of the PTHrP. For example, the fragment of the antibody includes Fab, F(ab')$_2$, Fv, or a single chain Fv (scFv) composed of a H-chain Fv fragment or a L-chain Fv fragment linked together through a suitable linker. Specifically, such antibody fragments can be produced by cleaving the antibody with an enzyme (e.g., papain, pepsin) into antibody fragments, or by constructing a gene encoding the antibody fragment and inserting the gene into an expression vector and introducing the resultant recombinant expression vector into a suitable host cell, thereby expressing the antibody fragment (see, for example, Co, M. S., et al., J. Immunol. (1994), 152, 2968-2976; Better, M. & Horwitz, A. H., Methods in Enzymology (1989), 178, 476-496, Academic Press, Inc.; Plueckthun, A. & Skerra, A., Methods in Enzymology (1989) 178, 476-496, Academic Press, Inc.; Lamoyi, E., Methods in Enzymology (1989) 121, 652-663; Rousseaux, J. et al., Methods in Enzymology (1989) 121, 663-669; and Bird, R. E. et al., TIBTECH (1991) 9, 132-137).

A scFv can be produced by ligating the H-chain V-region to the L-chain V-region through a linker, preferably a peptide linker (Huston, J. S. et al., Proc. Natl. Acad. Sci. USA (1988) 85, 5879-5883). The H-chain V-region and the L-chain V-region in the scFv may be derived from any one of the antibodies described herein. The peptide linker which binds the V-regions may be any single chain peptide, for example, of 12-19 amino acid residues.

The DNA encoding the scFv can be prepared by first amplifying the DNA encoding the H-chain V-region and the DNA encoding the L-chain V-region of the antibody separately using a DNA fragment encoding the entire region of the H-chain or a portion thereof that includes the V-region and a DNA fragment encoding the entire region of the L-chain or a portion thereof that includes the V-region as templates and primer pairs that define the terminal ends of the DNA fragments; and then amplifying the DNA encoding the peptide linker using a DNA fragment encoding the peptide linker as a template and a primer pair that define the terminal ends of the DNA fragment so that the terminal ends of the peptide linker are ligated to the H-chain V-region and the L-chain V-region, respectively.

Once the DNA encoding the scFv is prepared, an expression vector carrying the DNA and a host transformed with the expression vector can be prepared by conventional methods. The scFv can be produced from the transformed host in any conventional method.

The antibody fragments used in the present invention may be produced by preparing genes for the fragments and expressing the genes in suitable hosts as described above. These antibody fragments are also encompassed in the "antibody" of the present invention.

As a modified form of the above-mentioned antibodies, for example, an anti-PTHrP antibody conjugated to any molecule (e.g., polyethylene glycol) may also be used. Such a modified antibody is also encompassed in the "antibody" of the present invention. The modified antibodies can be prepared by chemical modifications of the antibodies. The chemical modification techniques suitable for this purpose have already been established in the art.

6. Expression and production of recombinant antibody or modified antibody

The antibody gene constructed as described above can be expressed and obtained by known methods. For the expression in a mammalian cell, a conventional useful promoter, the antibody gene to be expressed and a poly(A) signal (located downstream to the 3' end of the antibody gene) are operably linked. For example, as the useful promoter/enhancer system, a human cytomegalovirus immediate early promoter/enhancer system may be used.

Other promoter/enhancer systems, for example, those derived from viruses (e.g., retrovirus, polyoma virus, adenovirus and simian virus 40 (SV40)) and those derived from mammalian cells (e.g., human elongation factor 1α (HEF1α)), may also be used for the expression of the antibody in the present invention.

When SV40 promoter/enhancer system is used, the gene expression may be performed readily by the method of Mulligan et al. (Nature (1979) 277, 108). When HEF1α promoter/enhancer system is used, the gene expression may be performed readily by the method of Mizushima et al. (Nucleic Acids Res. (1990) 18, 5322).

For the expression in *E. coli,* a conventional useful promoter, a signal sequence for secreting the antibody of interest and the antibody gene may be operably linked. As the promoter, *lacZ* promoter or *araB* promoter may be used. When *lacZ* promoter is used, the gene expression may be performed by the method of Ward et al. (Nature (1098) 341, 544-546; FASEB J. (1992) 6, 2422-2427), while when *araB* promoter is used, the gene expression may be performed by the method of Better et al. (Better et al., Science (1988) 240, 1041-1043).

With respect to the signal sequence for secretion of the antibody, when the antibody of interest is intended to be secreted in a periplasmic space of the *E. coli, pelB* signal sequence (Lei, S. P. et al., J. Bacteriol. (1987) 169, 4379) may be used. The antibody secreted into the periplasmic space is isolated and then refolded so that the antibody takes an appropriate configuration.

The replication origin derived from viruses (e.g., SV40, polyoma virus, adenovirus, bovine papilloma virus (BPV)) or the like may be used. In order to increase the gene copy number in the host cell system, the expression vector may additionally contain a selective marker gene, such as an aminoglycoside phosphotranferase (APH) gene, a thymidine kinase (TK) gene, an *E. coli* xanthine-guanine phosphoribosyltransferase (Ecogpt) gene and a dihydrofolate reductase (dhfr) gene.

For the production of the antibody used in the present invention, any expression system including eukaryotic and prokaryotic cell systems may be used. The eukaryotic cell includes established cell lines of animals (e.g., mammals, insects, molds and fungi, yeast). The prokaryotic cell includes bacterial cells such as *E. coli* cells.

It is preferable that the antibody used in the present invention be expressed in a mammalian cell, such as a CHO, COS, myeloma, BHK, Vero and HeLa cell.

Next, the transformed host cell is cultured *in vitro* or *in vivo* to produce the antibody of interest. The cultivation of the host cell may be performed by any known method. The culture medium usable in the present invention may be DMEM, MEM, RPMI 1640 or IMDM medium. The culture medium may contain a serum supplement, such as fetal calf serum (FCS).

7. Isolation and purification of antibody

The antibody expressed and produced as described above may be isolated from the cells or the host animal body and purified to uniformity. The isolation and purification of the antibody used in the present invention may be performed on an affinity column. Examples of a protein A column include Hyper D, POROS and Sepharose F.F. (Pharmacia). Other methods conventionally used for the isolation and purification of an antibody may be also be used; thus the method is not particularly limited. For example, various chromatographs using columns including the above-mentioned affinity column, filtration, ultrafiltration, salting out and dialysis may be used singly or in combination to isolate and purify the antibody of interest (Antibodies A Laboratory Manual. Ed. Harlow, David Lane, Cold Spring Harbor Laboratory, 1988).

8. Determination of the activities of the antibody

The determination of the antigen-binding activity (Antibodies A Laboratory Manual, Ed. Harlow, David Lane, Cold Spring Harbor Laboratory, 1988) or the inhibitory activity against a ligand receptor (Harada, A. et al., International Immunology (1993) 5, 681-690) of the antibody used in the present invention may be performed by any known methods.

As the method for the determination of the antigen-binding activity of the anti-PTHrP antibody used in the present invention, there may be employed, for example, ELISA (enzyme-linked immunosorbent assay), EIA (enzyme immunoassay), RIA (radioimmunoassay) or fluorescent antibody technique. For example, when enzyme immunoassay is employed, a sample solution containing the anti-PTHrP antibody (e.g., a culture supernatant of anti-PTHrP antibody-producing cells, or the anti-PTHrP antibody *per se* in a purified form) is added to a plate on which PTHrP (1-34) is previously coated. A secondary antibody labeled with an enzyme (e.g., alkaline phosphatease) is further added to the plate. The plate is incubated and washed. A substrate for the enzyme (e.g., p-nitropbenylphosphoric acid) is added to the plate, and the absorbance of the solution in the plate is measured to evaluate the antigen-binding activity of the antibody.

To confirm the activity of the antibody used in the present invention, a neutralizing activity of the antibody (e.g., anti-PTHrP antibody) is determined.

9. Routes for administration and pharmaceutical preparations

The therapeutic agent of the present invention can be used for treatment or amelioration of hypercalcemic crisis associated with malignant tumor.

An effective single dose may be selected within the range from 0.001 to 1,000 mg per kg of body weight. Alternatively, the dose to a patient may be selected within the range from 0.01 to 100,000 mg/body, preferably 0.1 to 10,000 mg/body, more preferably 0.5 to 1,000 mg/body, still more preferably 1 to 100 mg/body. However, the dose of the therapeutic agent comprising the anti-PTHrP antibody of the present invention is not particularly limited to the above-mentioned ranges.

The therapeutic agent may be administered to a patient at any stage, including before or after the development of the hypercalcemic crisis associated with malignant tumor. Alternatively, the therapeutic agent may be administered at a stage where the development of weight loss is predicted in the patient.

The therapeutic agent comprising the anti-PTHrP antibody as an active ingredient of the present invention may be formulated by any conventional method (Remington's Pharmaceutical Science, latest edition, Mark Publishing Company, Easton, USA). The formulation may further comprise pharmaceutically acceptable carriers and additives.

Examples of such carriers and additives include water, pharmaceutically acceptable organic solvents, collagen, polyvinyl alcohol, polyvinyl pyrrolidone, carboxyvinyl polymer, sodium carboxymethyl cellulose, poly(sodium acrylate), sodium arginate, water soluble dextran, sodium carboxymethyl starch, pectin, methyl cellulose, ethyl cellulose, xanthane gum, gum arabic, casein, agar, polyethylene glycol, diglycerin, glycerin, propylene glycol, vaseline, paraffin, stearyl alcohol, stearic acid, human serum albumin (HSA), mannitol, sorbitol, lactose, and surfactants acceptable as pharmaceutical additives.

In the practical use, the additive is properly selected from the above members either singly or in combination depending on the dosage form employed, but not limited thereto. For example, an injection may be used which is prepared by dissolving the anti-PTHrP antibody in a purified form into a solvent (e.g., physiological saline, a buffer, a grape sugar solution) and then further adding an adsorption-preventing agent (e.g., Tween 80, Tween 20, a gelatin, human serum albumin) thereto. The therapeutic agent of the present invention may also be in a re-constitutive, freeze-dried form, which is dissolved before use. For the preparation of the freeze-dried dosage form, an excipient such as a sugar alcohol (e.g., mannitol, grape sugar) and a sugar may be incorporated.

BRIEF DESCRIPTION OF DRAWINGS

Fig. 1 is a graphical illustration of the therapeutic effect (in terms of the variation of blood ionized calcium level) of a murine anti-PTHrP antibody on hypercalcemic crisis associated with malignant tumor.

Fig. 2 is a graphical illustration of the therapeutic effect (in terms of the variation of body weight) of a murine anti-PTHrP antibody on hypercalcemic crisis associated with malignant tumor.

Fig. 3 is a graphical illustration of the therapeutic effect (in terms of the variation of blood ionized calcium level) of a humanized anti-PTHrP antibody on hypercalcemic crisis associated with malignant tumor.

Fig. 4 is a graphical illustration of the therapeutic effect (in terms of the variation of body weight) of a humanized anti-PTHrP antibody on hypercalcemic crisis associated with malignant tumor.

Fig. 5 is a graphical illustration of the therapeutic effect (in terms of the variation of blood ionized calcium level) of a humanized anti-PTHrP antibody on hypercalcemic crisis associated with malignant tumor.

Fig. 6 is a graphical illustration of the therapeutic effect (in terms of the variation of body weight) of a humanized anti-PTHrP antibody on hypercalcemic crisis associated with malignant tumor.

EXAMPLES

Hereinbelow, the present invention will be described in greater detail with reference to the following Reference Examples and Examples, which should not be construed as limiting the technical scope of the invention.

[EXAMPLE 1] Pharmacological test on model animal with hypercalcemic crisis associated with malignant tumor

Using a hypercalcemia model animal (a human tumor-transplanted nude mouse), a murine monoclonal antibody and a humanized monoclonal antibody both against PTHrP were examined for the therapeutic effect on hypercalcemic crisis.
As model animals, nude mice or rats transplanted with human pancreatic cancer strain FA-6 [given by Professor Kanji Satoh, Tokyo Women's Medical University] or human lung cancer strain LC-6-JCK [purchased from the Central Institute for Experimental Animals] were used. It has been known that nude mice or rats transplanted with such human tumor strains exhibit a rapid increase in blood calcium level as increasing the tumor volume and, as a result, develop so-called "hypercalcemic crisis" including as rapid weight loss and worsening of general condition which in some cases results in death. In this test, amelioration of such conditions by the murine monoclonal antibody and the humanized monoclonal antibody was evaluated by determining body weight and blood calcium level as measures.

Passage and maintaining of human tumor strain and preparation of model animals

Passage of human pancreatic cancer strain FA-6 and human lung cancer strain LC-6 was made *in vivo* using BALB/c-nu/nu nude mice (CLEA Japan, Inc.). For the evaluation of pharmacological effect on mice, 5-weeks-old male BALB/c-nu/nu nude mice (BALB/cAJcl-nu; CLEA Japan, Inc.) were purchased and acclimatized for 1 week to give 6-weeks-old mice for use in the evaluation. For the evaluation of pharmacological effect on rats, 5-weeks-old male nude rats (F344/NJcl-rnu; CLEA Japan, Inc.) were purchased and acclimatized for 1 week to give 6-weeks-old mice in the same manner as for the mice, which were provided for use in the evaluation.
The hypercalcemia model animals were prepared and divided into groups in the following manner. The passaged tumor was removed, and then finely cut into 3-mm cube of blocks. The resultant tumor blocks were subcutaneously transplanted into each of the animals at the lateral region at one piece per animal. When it was confirmed that the tumor volume became sufficiently large and the blood ionized calcium level is increased in each of the animals, the animals were divided into groups so that the tumor volumes, the blood calcium levels and the body weights of the animals in the individual groups were averaged, which were provided for use as the hypercalcemia model animals. The mice and rats were divided into three groups, respectively; a test group to which the antibody to be examined is administered; a test group to which a control agent is administered; and a control group to which a solvent (in this test, phosphate-buffered saline (PBS)) is administered.

Determination of blood calcium level

Two, four and twenty-four hours (in the pharmacological test for the mice) or one, two, four and twenty-four hours (in the pharmacological test for the rats) after the administration of each of the antibodies, the blood calcium level of each of the animals was determined to evaluate the pharmacological efficacy of the antibodies. The blood calcium level was determined as a whole blood ionized calcium level, by drawing blood from each of the animals via the orbit (for the mice) or the tail vein (for the rats) using a hematocrit tube and applying the blood to 643 Automatic Ca/pH Analyzer (CIBA-CORNING). The body weight of each animal was weighed before and 24 hours after the administration of the antibody.

Determination of tumor volume

The tumor volume was determined by measuring the longest axis (a mm) and the shortest axis (b mm) of the tumor and applying the both measured values to Galant's equation [$ab^2/2$].
The examination of the therapeutic effect on hypercalcemic crisis associated with malignant tumor was performed in the following manner.

(1) Pharmacological test for murine monoclonal antibody (#23-57-137-1)

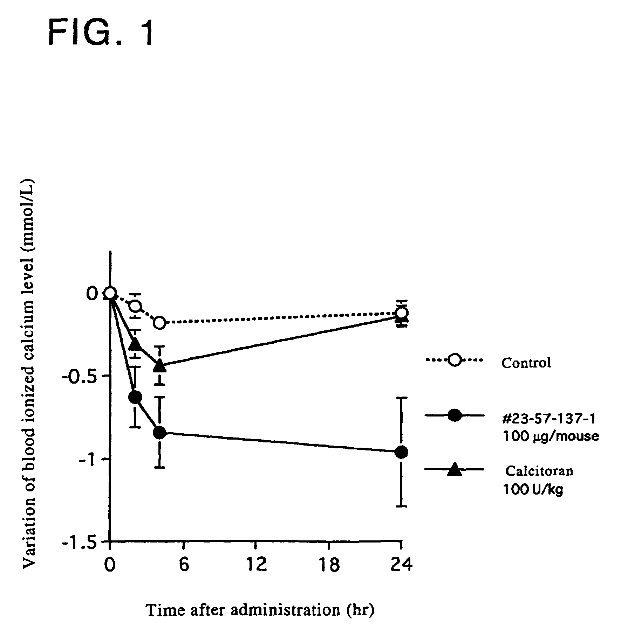

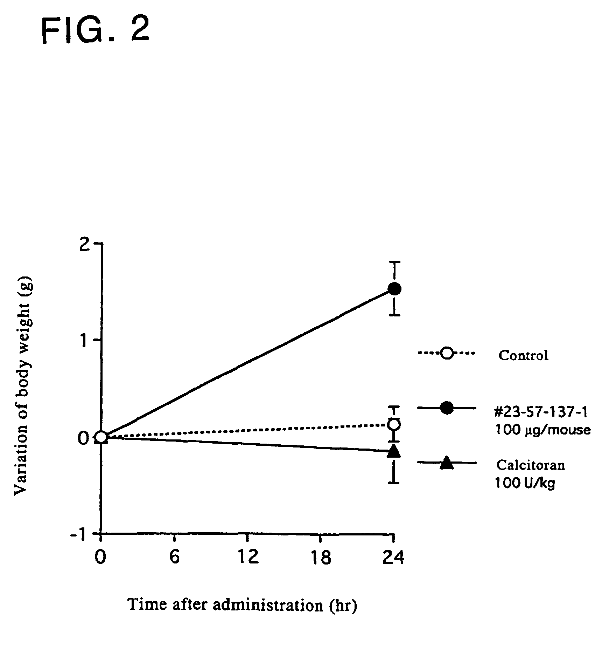

In the pharmacological test for the murine monoclonal antibody, 100 µg of the antibody which had been dissolved in 0.1 mL of PBS was administered to the model mice of a test group (which had been prepared and divided into the groups as mentioned above) once via the tail vein at a dose of 100 µg/0.1 mL PBS/mouse. As a control agent, a calcitonin preparation ("CALCITORAN" from Teikoku Hormone Mfg. Co., Ltd.), which is an existing hypercalcemic crisis-treating agent, was administered to the mice of another test group once via the tail vein at a dose of 100 U/kg. As a control, PBS was administered to the mice of a control group once via the tail vein at a dose of 0.1 mL/mouse. (See FIGs. 1 and 2)

(2) Pharmacological test for humanized antibody (hMBC(q))

1. Pharmacological test on hypercalcemic model mice

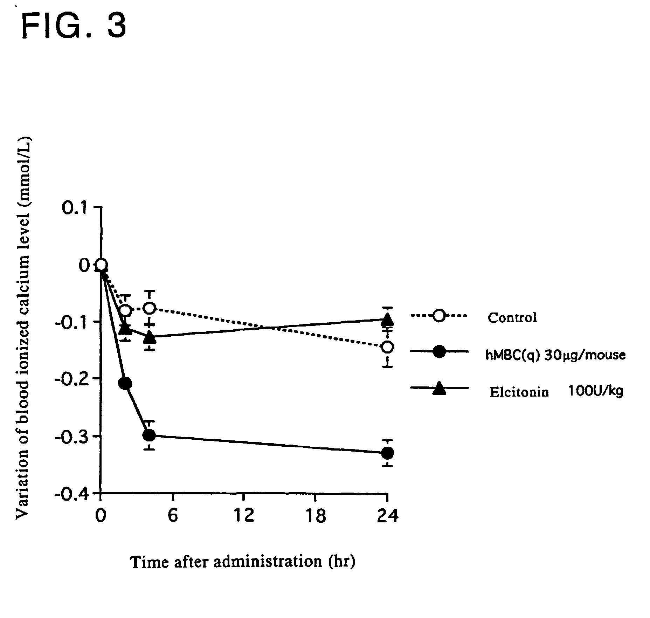

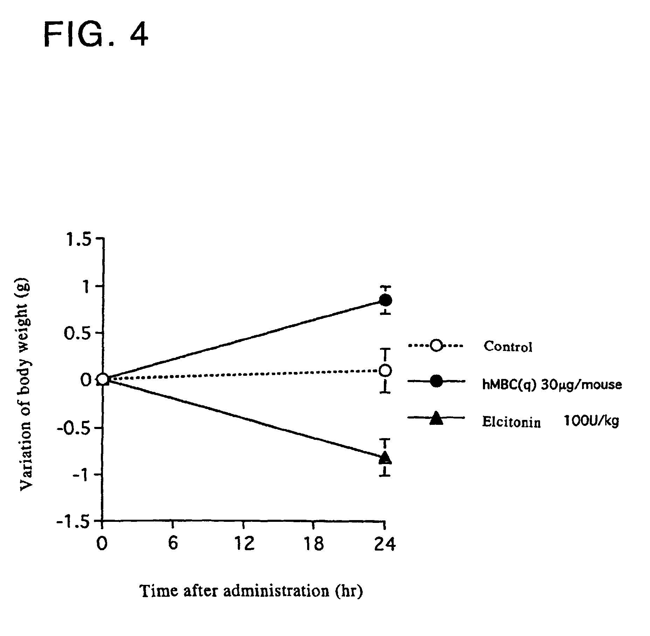

In the pharmacological test for the humanized antibody, the antibody was administered to the hypercalcemia model mice of a test group once via the tail vein at a dose of 30 µg/0. 1 mL PBS/mouse. As a control agent, a calcitonin preparation ("Elcitonin" from Asahi Chemical Industry Co., Ltd.) was administered to the mice of another test group once via the tail vein at a dose of 100 U/kg. As a control, PBS was administered to the mice of a control group once via the tail vein at a dose of 0.1 mL/mouse. (See FIGs. 3 and 4)

2. Pharmacological test on hypercalcemic model rats

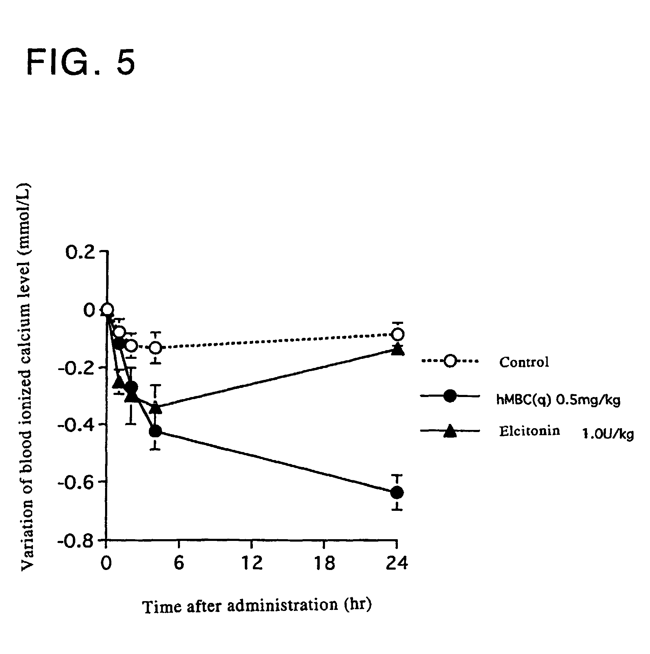

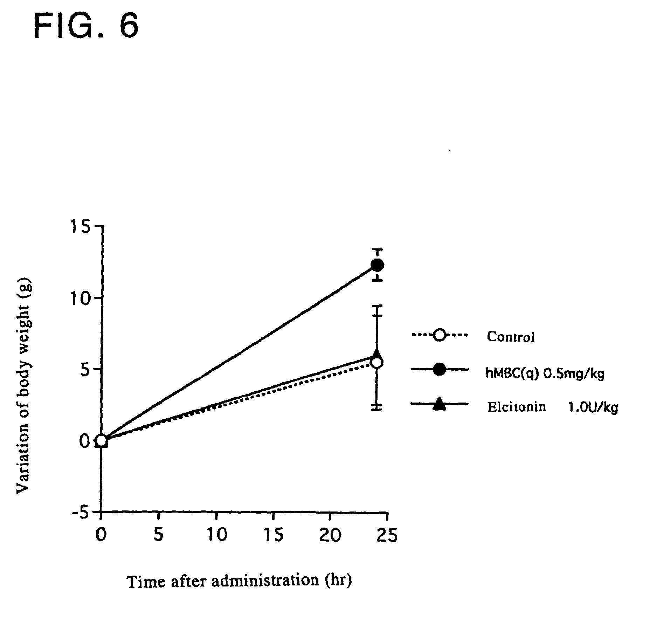

In the pharmacological test for the humanized antibody, the antibody was administered to the hypercalcemia model rats of a test group once via the tail vein at a dose of 0.5 mg/l mL PBS/kg. As a control agent, a calcitonin preparation ("Elcitonin" from Asahi Chemical Industry Co., Ltd.) was administered to the rats of another test group once via the tail vein at a dose of 1 U/kg. As a control, PBS was administered to the rats of a control group once via the tail vein at a dose of 1 mL/mouse. (See FIGs. 5 and 6)

As apparent from the results, the murine monoclonal antibody and the humanized antibody rapidly decreased the blood ionized calcium levels of the hypercalcemia model animals (FIGs. 1, 3 and 5). The antibodies exerted rapid onset and a long duration of action, although the action of the calcitonin preparation was temporary. In the antibody-administered groups, recovery of body weight was observed 24 hours after the administration, which was not observed in the calcitonin preparation-administered groups (FIGs. 2, 4 and 6). These results clearly demonstrate that the murine monoclonal antibody or the humanized antibody which has a neutralizing activity against PTHrP is useful as a therapeutic agent for hypercalcemic crisis associated with malignant tumor for which emergency treatment must be considered.

[REFERENCE EXAMPLE 1]

Preparation of hybridomas producing anti-PTHrP (1-34) murine monoclonal antibody

Hybridomas capable of producing a monoclonal antibody against human PTHrP (1-34) #23-57-154 and #23-57-137-1 were prepared in the following manner (Sato, K. et al., J. Bone Miner. Res. 8, 849-860, 1993). The amino acid sequence of human PTHrP is shown in SEQ ID NO: 75.

The immunogen used was PTHrP (1-34) (Peninsula), to which a carrier protein thyroglobulin was conjugated with carbodiimide (Dojinn). The thycloglobulin-conjugated PTHrP (1-34) was dialyzed to obtain a solution having a protein concentration of 2 µg/mL. The resultant solution was mixed with Freund's adjuvant (Difco) at a mixing ratio of 1:1 to give an emulsion. This emulsion was injected to 16 female BALB/C mice 11 times dorsal-subcutaneously or intraperitoneally at a dose of 100 µg/mouse for each injection, thereby immunizing the mice. For the priming immunization, Freund's complete adjuvant was used; while for the boosting immunization, Freund's incomplete adjuvant was used.

Each of the immunized mice was determined for its antibody titer in the serum in the following manner. That is, each of the mice was blood-drawn via its tail vein, and the anti-serum is separated from the blood. The anti-serum was diluted with a RIA buffer and mixed with $^{125}$I-labeled PTHrP (1-34) to determine the binding activity. The mice that were confirmed to have a sufficiently increased titer were injected with PTHrP (1-34) without a carrier protein intraperitoneally at a dose of 50 µg/mouse for the final immunization.

Three days after the final immunization, the mouse was sacrificed and the spleen was removed therefrom. The spleen cells were subjected to cell fusion with mouse myeloma cell line P3x63Ag8U.1 in accordance with any conventional known method using 50% polyethylene glycol 4000. The fused cells thus prepared were seeded to each well of 85 96-well plates at $2 \times 10^4$/well. Hybridomas were screened in HAT medium as follows.

The screening of hybridomas was performed by determining the presence of PTHrP-recognition antibodies in the culture supernatant of the wells in which cell growth had been observed in HAT medium, by a solid phase RIA method. The hybridomas were collected from the wells in which the binding ability to the PTHrP-recognition antibodies had been confirmed. The hybridomas thus obtained was suspended into RPMI-1640 medium containing 15% FCS supplemented with OPI-supplement (Sigma), followed by unification of the hybridomas by a limiting dilution method. Thus, two types of hybridoma clones, #23-57-154 and #23-57-137-1, could be obtained, both which had a strong binding ability to PTHrP (1-34).

Hybridoma clone #23-57-137-1 was designated "mouse-mouse hybridoma #23-57-137-1", and has been deposited under the terms of the Budapest Treaty on August 15, 1996 at the National Institute of Bioscience and Human-technology, Agency of Industrial Science and Technology, Japan (1-3, Higashi 1-chome, Tsukuba-shi, Ibaraki, Japan) under the accession No. FERM BP-5631.

[REFERENCE EXAMPLE 2]

Cloning of DNA encoding V-region of murine monoclonal antibody against human PTHrP (1-34)

Cloning of DNA encoding the V-region of a murine monoclonal antibody against human PTHrP (1-34) #23-57-137-1 was performed in the following manner.

(1) Preparation of mRNA

mRNA from hybridoma #23-57-137-1 was prepared using Quick Prep mRNA Purification Kit (Pharmacia Biotech). That is, cells of hybridoma #23-57-137-1 were fully homogenized with an extraction buffer, and mRNA was isolated and purified therefrom on an oligo(dT)-Cellulose Spun Column in accordance with the instructions included in the kit. The resultant solution was subjected to ethanol precipitation to obtain the mRNA as a precipitate. The mRNA precipitate was dissolved in an elution buffer.

(2) Production and amplification of cDNA for gene encoding mouse H-chain V-region

(i) Cloning of cDNA for #23-57-137-1 antibody H-chain V-region

A gene encoding H-chain V-region of the murine monoclonal antibody against human PTHrP was cloned by a 5'-RACE method (Frohman, M. A. et al., Proc. Natl. Acad. Sci. USA, 85, 8998-9002, 1988; Belyavsky, A. et al., Nucleic Acids Res. 17, 2919-2932, 1989). The 5'-RACE method was performed using 5'-Ampli FINDER RACE Kit (CLONETECH) in accordance with the instructions included in the kit. In this method, the primer used for synthesis of cDNA was MHC2 primer (SEQ ID NO: 1) which is capable of hybridizing to mouse H-chain C-region. The above-prepared mRNA (about 2 µg), which was a template for the cDNA synthesis, was mixed with MHC2 primer (10 pmoles). The resultant mixture was reacted with a reverse transcriptase at 52°C for 30 minuets to effect the reverse transcription of the mRNA into cDNA.

The resultant reaction solution was added with 6N NaOH to hydrolyze any RNA remaining therein (at 65°C for 30 min.) and then subjected to ethanol precipitation to isolate and purify the cDNA as a precipitate. The purified cDNA was ligated to Ampli FINDER Anchor (SEQ ID NO: 42) at the 5' end by reacting with T4 RNA ligase at 37°C for 6 hours and additionally at room temperature for 16 hours. As the primers for amplification of the cDNA by a PCR method, Anchor primer (SEQ ID NO: 2) and MHC-G1 primer (SEQ ID NO: 3) (S.T. Jones, et al., Biotechnology, 9, 88, 1991) were used.

The PCR solution comprised (per 50 µl) 10 mM Tris-HCl (pH 8.3), 50 mM KCl, 0.25 mM dNTPs (dATP, dGTP, dCTP, dTTP), 1.5 mM MgCl₂, 2.5 units of TaKaRa Taq (Takara Shuzo Co., Ltd.), 10 pmoles Anchor primer, and 1 µl of the reaction mixture of the cDNA to which MHC-G1 primer and Ampli FINDER Anchor primer had been ligated, over which mineral oil (50 µl) was layered. PCR was performed in Thermal Cycler Model 480J (Perkin Elmer) for 30 cycles under the conditions: 94°C for 45 sec.; 60°C for 45 sec.; and 72°C for 2 min.

(ii) Cloning of cDNA for #23-57-137-1 antibody L-chain V-region

A gene encoding L-chain V-region of the murine monoclonal antibody against human PTHrP was cloned by the 5'-RACE method (Frohman, M. A. et al., Proc. Natl. Acad. Sci. USA, 85, 8998-9002, 1988; Belyavsky, A. et al., Nucleic Acids Res. 17, 29 19-2932, 1989). The 5'-RACE method was performed using 5'-Ampli Finder RACE Kit (Clonetech) in accordance with the instructions included in the kit. In this method, oligo-dT primer was used as the primer for synthesizing cDNA. The above-prepared mRNA (about 2 µg), which was a template for the cDNA synthesis, was mixed with oligo-dT primer. The resultant mixture was reacted with a reverse transcriptase at 52°C for 30 min. to effect the reverse transcription of the mRNA into cDNA. The resultant reaction solution was added with 6N NaOH to hydrolyze any RNA remaining therein (at 65°C for 30 min.). The resultant solution was subjected to ethanol precipitation to isolate and purified the cDNA as a precipitate. The cDNA thus synthesized was ligated to Ampli FINDER Anchor at the 5' end by reacting with T4 RNA ligase at 37°C for 6 hours and additionally at room temperature for 16 hours.

A PCR primer MLC (SEQ ID NO: 4) was designed based on the conserved sequence of mouse L-chain λ chain C-region and then synthesized using 394 DNA/RNA Synthesizer (ABI). The PCR solution comprised (per 100 μl) 10 mM Tris-HCl (pH 8.3), 50 mM KCl, 0.25 mM dNTPs (dATP, dGTP, dCTP, dTTP), 1.5 mM MgCl$_2$, 2.5 units of AmpliTaq (PERKIN ELMER), 50 pmoles of Anchor primer (SEQ ID NO: 2), and 1 μl of the reaction mixture of the cDNA to which MLC (SEQ ID NO: 4) and Ampli FINDER Anchor were ligated, over which mineral oil (50 μl) was layered. PCR was performed in Thermal Cycler Model 480J (Perkin Elmer) for 35 cycles under the conditions: 94°C for 45 sec.; 60°C for 45 sec.; and 72° C for 2 min.

(3) Purification and fragmentation of PCR products

Each of the DNA fragments amplified by the PCR methods described above was separated by agarose gel electrophoresis on a 3% Nu Sieve GTG agarose (FMC Bio. Products). For each of the H-chain V-region and the L-chain V-region, an agarose gel segment containing a DNA fragment of about 550 bp was excised from the gel. Each of the gel segments was subjected to purification of the DNA fragment of interest using GENECLEAN II Kit (BIO101) in accordance with the instructions included in the kit. The purified DNA was precipitated with ethanol, and the DNA precipitate was dissolved in 20 μl of a solution containing 10 mM Tris-HCl(pH 7.4) and 1 mM EDTA. A portion (1 μl) of the DNA solution was digested with a restriction enzyme XmaI (New England Biolabs) at 37°C for 1 hour and further digested with a restriction enzyme EcoRI (Takara Shuzo Co., Ltd.) at 37°C for 1 hour. The digestion solution was extracted with phenol and chloroform and then precipitated with ethanol to collect the DNA.

In this manner, two DNA fragments containing a gene encoding mouse H-chain V-region and a gene encoding mouse L-chain V-region, respectively, were obtained, both which had an EcoRI recognition sequence on the 5' end and an XmaI recognition sequence on the 3' end.

The EcoRI-XmaI DNA fragments containing a gene encoding mouse H-chain V-region and a gene encoding mouse L-chain V-region, respectively, were separately ligeted to pUC19 vector that had been digested with EcoRI and XmaI at 16°C for 1 hour using DNA Ligation Kit ver.2 (Takara Shuzo Co., Ltd.) in accordance with the instructions included in the kit. A portion (10 μl) of the ligation mixture was added to 100 μl of a solution containing competent cells of *E. coli,* JM 109 (Nippon Gene Co., Ltd.). The cell mixture was allowed to stand on ice for 15 min., at 42°C for 1 min. and additionally for 1 min. on ice. The resultant cell mixture was added with 300 μl of SOC medium (Molecular Cloning: A Laboratory Manual, Sambrook, et al., Cold Spring Harbor Laboratory Press, 1989) and then incubated at 37°C for 30 min. The resultant cell solution was plated on LB agar medium or 2xYT agar medium (Molecular Cloning: A Laboratory Manual, Sambrook, et al., Cold Spring Harbor Laboratory Press, 1989) containing either 100 or 50 μg/mL of ampicillin, 0.1 mM of IPTG and 20 μg/mL of X-gal, and then incubated at 37°C overnight. In this manner, *E. coli* transformants were prepared.

The transformants were cultured at 37°C overnight in 2 mL of LB or 2xYT medium containing either 100 or 50 μg/mL of ampicillin. The cell fraction was applied to Plasmid Extracter PI-100Σ (Kurabo Industries, Ltd.) or QIAprep Spin Plasmid Kit (QIAGEN) to give plasmid DNA. The plasmid DNA thus obtained was sequenced.

(4) Sequencing of gene encoding mouse antibody V-region

The nucleotide sequence of the cDNA coding region carried on the plasmid was determined on DNA Sequencer 373A (ABI; Perkin-Elmer) using Dye Terminator Cycle Sequencing Kit (Perkin-Elmer). In this sequencing, M13 Primer M4 (Takara Shuzo Co., Ltd.) (SEQ ID NO: 5) and M13 Primer RV (Takara Shuzo Co., Ltd.) (SEQ ID NO: 6) were used, and the nucleotide sequence was confirmed in the both directions.

The plasmid containing a gene encoding mouse H-chain V-region derived from hybridoma #23-57-137-1 was designated "MBC1H04", and plasmid containing a gene encoding mouse L-chain V-region derived from hybridoma #23-57-137-1 was designated "MBC1L24". The nucleotide sequences (including the corresponding amino acids sequences) of the DNA encoding the mouse #23-57-137-1 antibody-derived H-chain V-region in plasmid MBC1H04 and gene encoding the mouse #23-57-137-1 antibody-derived L-chain V-region in plasmid MBC1H24 were shown in SEQ. ID Nos: 57 and 65, respectively. The amino acid sequences of the polypeptides for the H-chain V-region and the L-chain V-region were also shown in SEQ. ID NOs: 46 and 45, respectively.

The *E. coli* strain containing plasmid MBC1H04 and the *E. coli* strain containing plasmid MBC1L24 were designated "*Escherichia coli* JM109 (MBC1H04)" and "Escherichia coli JM109 (MBC1L24)", respectively. These *E. coli* strains have been deposited under the terms of the Budapest Treaty at the National Institute of Bioscience and Human-Technology, Agency of Industrial Science and Technology, Japan (1-3, Higashi 1-chome, Tsukuba-shi, Ibaraki, Japan) on August 15, 1996, under the Accession No. FERM BP-5628 for *Escherichia coli* JM109 (MBC1H04) and FERM BP-5627 for *Escherichia coli* JM109 (MBC1L24), respectively.

(5) Determination of CDRs of murine monoclonal antibody #23-57-137-1 against human PTHrP

The H-chain V-region and the L-chain V-region have general structures similar to each other, in which there are four framework regions (FRs) linked through three hypervariable regions (i.e., complementarity determining regions; CDRs). The amino acid sequences of the FRs are relatively well conserved, while the amino acid sequence of the CDRs have an extremely high variability (Kabat, E.A. et al., "Sequence of Proteins of Immunological Interest", US Dept. Health and Human Services, 1983).

In view of these facts, the homology in amino acid between the V-regions of the murine monoclonal antibody against human PTHrP was determined with reference to the database of amino acid sequences for antibodies established by Kabat et al. Thus, the CDRs of the V-regions were determined as shown in Table 1.

The amino acid sequences for CDRs 1-3 in the L-chain V-region shown in SEQ ID Nos: 59 to 61, respectively; and the amino acid sequences for CDRs 1-3 in the H-chain V-region are shown in SEQ ID Nos: 62 to 64, respectively.

Table 1

| V-region | SEQ ID NO. | CDR1 | CDR2 | CDR3 |
|---|---|---|---|---|
| H-chain V-region | 57 | 31-35 | 50-66 | 99-107 |
| L-chain V-region | 65 | 23-34 | 50-60 | 93-105 |

[REFERENCE EXAMPLE 3] Construction of Chimeric Antibody

(1) Construction of chimeric antibody H-chain

(i) Construction of H-chain V-region

To ligate to an expression vector carrying a genomic DNA of human H-chain C-region Cγ1, the cloned DNA encoding mouse H-chain V-region was modified by a PCR method. A backward primer MBC1-S1 (SEQ ID NO: 7) was designed to hybridize to a DNA sequence encoding the 5' region of the leader sequence for the V-region and to have both a Kozak consensus sequence (Kozak, M. et al., J. Mol. Biol., 196, 947-950, 1987) and a HindIII-recognition sequence. A forward primer MBC1-a (SEQ ID NO: 8) was designed to hybridize to a DNA sequence encoding the 3' region of the J region and to have both a donor splice sequence and a BamHI-recognition sequence. PCR was performed using TaKaRa Ex Taq (Takara Shuzo Co., Ltd.) and a buffer appended thereto. The PCR solution comprised (per 50 μl) 0.07 μg of plasmid MBC1H04 as a template DNA, 50 pmoles of MBC1-a and 50 pmoles of MBC1-S1 as primers, 2.5U of TaKaRa Ex Taq and 0.25 mM dNTPs in the buffer, over which 50 μl of mineral oil was layered. The PCR was run for 30 cycles under the conditions: 94°C for 1 min.; 55°C for 1 min.; 72°C for 2 min. The DNA fragments thus amplified by the PCR method were separated by agarose gel electrophoresis on a 3% Nu Sieve GTG Agarose (FMC Bio. Products).

Then, an agarose gel segment containing a DNA fragment of 437 bp was excised, and the DNA fragment was purified therefrom using GENECLEAN II Kit (BIO101) in accordance with the instructions included in the kit. The purified DNA was collected by ethanol precipitation, and then dissolved in 20 μl of a solution containing 10 mM Tris-HCl (pH 7.4) and 1 mM EDTA. A portion (1 μl) of the resultant DNA solution was digested with restriction enzymes BamHI and HindIII (Takara Shuzo Co., Ltd.) at 37°C for 1 hour. The digestion solution was extracted with phenol and chloroform and then precipitated with ethanol to collect the DNA of interest.

The obtained HindIII-BamHI DNA fragment, which containing a gene encoding the mouse H-chain V-region, was subcloned into pUC19 vector that had been digested with HindIII and BamHI. The resultant plasmid was sequenced on DNA Sequencer 373A (Perkin-Elmer) using M13 Primer M4 and M13 Primer RV as primers and Dye Terminator Cycle Sequencing Kit (Perkin-Elmer). As a result, a plasmid which carried a gene of correct nucleotide sequence encoding the mouse H-chain V-region derived from hybridoma #23-57-137-1 and had a HindIII-recognition sequence and a Kozak sequence on its 5' region and a BamHI-recognition sequence on its 3' region was obtained, which was designated "MBC1H/pUC19".

(ii) Construction of H-chain V-region for cDNA-type of mouse-human chimeric H-chain

To ligate to cDNA of the human H-chain C-region Cγ1, the DNA encoding the mouse H-chain V-region constructed as described above was modified by a PCR method. A backward primer MBC1HVS2 (SEQ ID NO: 9) for the H-chain V-region was designed to cause the replacement of the second amino acid (asparagine) of the sequence encoding the front portion of the leader sequence for the H-chain V-region by glycine and to have a Kozak consensus sequence (Kozak, M. et al., J. Mol. Biol., 196, 947-950, 1987) and HindIII- and EcoRI-recognition sequences. A forward primer MBC1HVR2 (SEQ ID NO: 10) for the H-chain V-region was designed to hybridize to the DNA sequence encoding the 3' region of the J region, to encoding the 5' region of the C-region and to have ApaI- and SmaI-recognition sequences.

PCR was performed using TaKaRa Ex Taq (Takara Shuzo Co., Ltd.) and a buffer appended thereto. The PCR solution comprised (per 50 μl) 0.6 μg of plasmid MBC1H/pUC19 as a template DNA, 50 pmoles of MBC1HVS2 and 50 pmoles of MBC1HVR2 as primers, 2.5U of TaKaRa Ex Taq and 0.25mM of dNTPs in the buffer, over which 50 μl of mineral oil was layered. The PCR was run for 30 cycles under the conditions: 94°C for 1 min.; 55°C for 1 min.; 72°C for 1 min. The DNA fragments amplified by the PCR were separated by agarose gel electrophoresis on a 1% Sea Kem GTG Agarose (FMC Bio. Products). Then, an agarose gel segment containing a DNA fragment of 456 bp was excised and the DNA fragment was purified therefrom using GENECLEAN II Kit (BIO101) in accordance with the instructions included in the kit. The purified DNA was precipitated with ethanol and then dissolved in 20 μl of a solution containing 10 mM Tris-HCl (pH 7.4) and 1 mM EDTA.

The resultant DNA solution (1 μg) was digested with restriction enzymes EcoRI and SmaI (Takara Shuzo Co., Ltd.) at 37°C for 1 hour. The digestion solution was extracted with phenol and chloroform and then precipitated with ethanol to collect the DNA. The obtained EcoRI-SmaI DNA fragment, which containing a gene encoding the mouse H-chain V-region, was subcloned into pUC19 vector that had been digested with EcoRI and SmaI. The resultant plasmid was sequenced on DNA Sequencer 373A (Perkin-Elmer) using M13 Primer M4 and M13 Primer RV, and Dye Terminator Cycle Sequencing Kit (Perkin-Elmer). As a result, a plasmid which contained a gene encoding mouse H-chain V-region derived from hybridoma #23-57-137-1 of correct nucleotide sequence and had EcoRI- and HindIII-recognition sequences and a Kozak sequence on its 5' region and ApaI- and SmaI-recognition sequences on its 3' region was obtained, which was designated "MBC1Hv/pUC19".

(iii) Construction of expression vector for chimeric antibody H-chain

cDNA containing the DNA for human antibody H-chain C-region Cγ1 was prepared as follows. mRNA was prepared from a CHO cell into which both an expression vector DHFR-ΔE-RVh-PM-1-f (see WO 92/19759) encoding the genomic DNAs of humanized PM1 antibody H-chain V-region and human antibody H-chain C-region IgG1 (N. Takahashi et al., Cell 29, 671-679, 1982) and an expression vector RV1-PM1a (see WO 92/19759) encoding the genomic DNAs of humanized PM1 antibody L-chain V-region and human antibody L-chain κ chain C-region had been introduced. Using the mRNA, cDNA containing the humanized PM1 antibody H-chain V-region and the human antibody C-region Cγ1 was cloned by a RT-PCR method, and then subcloned into plasmid pUC19 on the HindIII-BamHI site. After sequencing, a plasmid which had the correct nucleotide sequence was obtained, which was designated "pRVh-PM1f-cDNA".

An expression vector DHFR-ΔE-RVh-PM-1-f in which both a HindIII site between SV40 promoter and a DHFR gene and an EcoRI site between EF-1α promoter and a humanized PM1 antibody H-chain V-region gene had been deleted, was prepared for the construction of an expression vector for cDNA containing the humanized PM1 antibody H-chain V-region gene and the human antibody C-region Cγ1 gene.

The plasmid obtained (pRVh-PM1f-cDNA) was digested with BamHI, blunt-ended with Klenow fragment, and further digested with HindIII, thereby obtaining a blunt-ended HindIII-BamHI fragment. The blunt-ended HindIII-BamHI fragment was ligated to the above-mentioned HindIII site- and EcoRI site-deleted expression vector DHFR-ΔE-RVh-PM1-f that had been digested with HindIII and BamHI. Thus, an expression vector RVh-PM1f-cDNA was constructed which contained cDNA encoding the humanized PM1 antibody H-chain V-region and the human antibody C-region Cγ1.

The expression vector RVh-PM1f-cDNA containing the cDNA encoding the humanized PM1 antibody H-chain V-region and the human antibody C-region Cγ1 was digested with ApaI and BamHI, and a DNA fragment containing the H-chain C-region was collected therefrom. The resultant DNA fragment was introduced into the above-mentioned plasmid MBC1Hv/pUC19 that had been digested with ApaI and BamHI. The plasmid thus prepared was designated "MBC1HcDNA/pUC19". This plasmid contained cDNA encoding the mouse antibody H-chain V-region and the human antibody C-region Cγ1, and had EcoRI- and HindIII-recognition sequences on its 5' region and a BamHI-recognition sequence on its 3' region.

The plasmid MBC1HcDNA/pUC19 was digested with EcoRI and BamHI to give a DNA fragment comprising a nucleotide sequence encoding the chimeric antibody H-chain. The resultant DNA fragment was introduced into an expression vector pCOS1 that had been digested with EcoRI and BamHI, thereby giving an expression vector for the chimeric antibody, which was designated "MBC1HcDNA/pCOS1". Here, the expression vector pCOS1 was constructed using HEF-PMh-gγ1 (see WO 92/19759) by deleting therefrom an antibody genes by digestion with EcoRI and SmaI, and then ligating it to EcoRI-NotI-BamHI Adaptor (Takara Shuzo Co., Ltd.).

For preparing a plasmid for the expression in a CHO cell, the plasmid MBC1HcDNA/pUC19 was digested with EcoRI and BamHI to obtain a DNA fragment containing a gene for the chimeric antibody H-chain. The DNA fragment was then introduced into an expression plasmid pCHO1 that had been digested with EcoRI and BamHI to give an expression plasmid for the chimeric antibody, which was designated "MBC1HcDNA/pCHO1". Here, the expression vector pCHO1 was constructed using DHFR-ΔE-rvH-PM1-f (see WO 92/19759) by deleting therefrom an antibody gene by digestion with EcoRI and SmaI, and then ligating it to EcoRI-NotI-BamHI Adaptor (Takara Shuzo Co., Ltd.).

(2) Construction of human L-chain C-region

(i) Preparation of cloning vector

To construct pUC19 vector containing a gene for human L-chain C-region, a HindIII site-deleted pUC19 vector was prepared. pUC19 vector (2 μg) was digested in 20 μl of a reaction solution containing 20 mM Tris-HCl (pH 8.5), 10 mM MgCl$_2$, 1 mM DTT, 100mM KCl, 8 U of HindIII (Takara Shuzo Co., Ltd.) at 37°C for 1 hour. The resultant digestion solution was extracted with phenol and chloroform, and then subjected to ethanol precipitation to collect the DNA of interest.

The DNA collected was reacted in 50 μl of a reaction solution containing 50 mM Tris-HCl (pH 7.5), 10 mM MgCl$_2$, 1 mM DTT, 100 mM NaCl, 0.5 mM dNTPs and 6U of Klenow fragment (GIBCO BRL) at room temperature for 20 min., thereby rendering the terminal ends of the DNA blunt. This reaction mixture was extracted with phenol and chloroform and then subjected to ethanol precipitation to collect the vector DNA.

The vector DNA thus collected was reacted in 10 μl of a reaction solution containing 50 mM Tris-HCl (pH 7.6), 10 mM MgCl$_2$, 1 mM ATP, 1 mM DTT, 5% (v/v) polyethylene glycol-8000 and 0.5 U of T4 DNA ligase (GIBCO BRL) at 16°C for 2 hours, to cause self-legation of the vector DNA. The reaction solution (5 μl) was added to 100 μl of a solution containing competent cells of *E. coli,* JM109 (Nippon Gene Co., Ltd.), and the resultant solution was allowed to stand on ice for 30 min., at 42°C for 1 min., and further on ice for 1 min. SOC culture medium (500 μl) was added to the reaction solution and then incubated at 37°C for 1 hour. The resultant solution was plated on 2xYT agar medium (containing 50 μg/mL of ampicillin) which had been applied with X-gal and IPTG on its surface (Molecular Cloning: A Laboratory Manual, Sambrook, et al., Cold Spring Harbor Laboratory Press, 1989), and then cultured at 37°C overnight, thereby obtaining a transformant.

The transformant was cultured in 2xYT medium (20 mL) containing ampicillin (50 μg/mL) at 37°C overnight. From the cell fraction of the culture medium, a plasmid DNA was isolated and purified using Plasmid Mini Kit (QIAGEN) in accordance with the instructions included in the kit. The purified plasmid was digested with HindIII. The plasmid that was confirmed to have a HindIII site-deletion was designated "pUC19 ΔHindIII".

(ii) Construction of DNA encoding human L-chain λ chain C-region

Human antibody L-chain λ chain C-region has been known to have at least four isotypes including Mcg$^+$Ke$^+$Oz$^-$, Mcg$^-$Ke$^-$Oz$^-$, Mcg$^-$Ke$^-$Oz$^+$ and Mcg$^-$Ke$^+$Oz$^-$ (P. Dariavach, et al., Proc. Natl. Acad. Sci. USA, 84, 9074-9078, 1987). A search was made for a human antibody L-chain λ chain C-region homologous to the #23-57-137-1 mouse L-chain λ chain C-region from the EMBL database. As a result, it was found that the isotype Mcg$^+$Ke$^+$Oz$^-$ of the human antibody L-chain λ chain (Accession No. X57819) (P. Dariavach, et al., Proc. Natl. Acad. Sci. USA, 84, 9074-9078, 1987) showed the highest degree of homology to the #23-57-137-1 mouse L-chain λ chain C-region, with a 64.4% homology in terms of amino acid sequence and a 73.4% homology in terms of nucleotide sequence.

Then, a gene encoding human antibody L-chain λ chain C-region was constructed by a PCR method. The primer for the PCR was synthesized using 394 DNA/RNA Synthesizer (ABI). The synthesized primers were as follows: HLAMB1 (SEQ ID NO: 11) and HLAMB3 (SEQ ID NO: 13), both having a sense DNA sequence; and HLAMB2 (SEQ ID NO: 12) and HLAMB4 (SEQ ID NO: 14), both having an antisense DNA sequence; each primer containing a complementary sequence of 20-23 bp on the both terminal ends.

External primers HLAMBS (SEQ ID NO: 15) and HLAMBR (SEQ ID NO: 16) had sequences homologous to the primers HLAMB1 and HLAMB4, respectively. HLAMBS contained EcoRI-, HindIII- and BlnI-recognition sequences, and HLAMBR contained an EcoRI-recognition sequence. In the first PCR, the reactions between HLAMB1 and HLAMB2 and between HLAMB3 and HLAMB4 were performed. After the reactions were completed, both of the resultant PCR products were mixed in equivalent quantities, and then assembled in the subsequent second PCR. The reaction solution was added with the external primers HLAMBS and HLAMBR. This reaction mixture was subjected to the third PCR to amplify the full length DNA.

Each PCR was performed using TaKaRa Ex Taq (Takara Shuzo Co., Ltd.) in accordance with the instructions included in the kit. In the first PCR, 100 μl of either a reaction solution containing 5 pmoles of HLAMB1, 0.5 pmole of HLAMB2 and 5U of TaKaRa Ex Taq (Takara Shuzo Co., Ltd.) or a reaction solution containing 0.5 pmole of HLAMB3, 5 pmoles of HLAMB4 and 5U of TaKaRa Ex Taq (Takara Shuzo Co., Ltd.) was used, over which 50 µl of mineral oil was layered. The PCR was run for 5 cycles under the conditions: 94°C for 1 min., 60°C for 1 min. and 72°C for 1 min.

In the second PCR, a mixture of both the reaction solutions (50 µl each) was used, over which 50 µl of mineral oil was layered. The PCR was run for 3 cycles under the conditions: 94°C for 1 min., 60°C for 1 min. and 72°C for 1 min.

In the third PCR, the reaction solution to which the external primers HLAMBS and HLAMBR (50 pmoles each) were added was used. The PCR was run for 30 cycles under the conditions: 94°C for 1 min., 60°C for 1 min. and 72°C for 1 min.

The DNA fragment obtained by the third PCR was subjected to electrophoresis on a 3% low-melting agarose gel (NuSieve GTG Agarose, FMC), and separated and purified from the gel using GENECLEAN II Kit (BIO101) in accordance with the instructions included in the kit.

The DNA fragment obtained was digested in a reaction solution (20 µl) containing 50 mM Tris-HCl (pH 7.5), 10 mM $MgCl_2$, 1 mM DTT, 100 mM NaCl and 8U of EcoRI (Takara Shuzo Co., Ltd.) at 37°C for 1 hour. The digestion solution was extracted with phenol and chloroform, and the DNA was collected therefrom by the ethanol precipitation. The DNA was dissolved in a solution (8 µl) containing 10 mM Tris-HCl (pH 7.4) and 1 mM EDTA.

The above-prepared plasmid pUC19 ΔHindIII (0.8 µg) was digested with EcoRI in the same manner as mentioned above. The digestion solution was subjected to phenol/chloroform extraction and then ethanol precipitation, thereby giving a digested plasmid pUC19 ΔHindIII. The digested plasmid was reacted in a reaction solution (50 µl) containing 50 mM Tris-HCl (pH 9.0), 1 mM $MgCl_2$ and alkaline phosphatase (E. coli C75; Takara Shuzo Co., Ltd.) at 37°C for 30 min. to dephosphorylate (i.e., BAP-treat) the plasmid. The reaction solution was subjected to phenol/chloroform extraction, and the DNA was collected therefrom by ethanol precipitation. The DNA thus obtained was dissolved in a solution (10 µl) containing 10 mM Tris-HCl (pH 7.4) and 1 mM EDTA.

The BAP-treated plasmid pUC19 ΔHindIII (1 µl) was ligated to the above-obtained PCR product (4 µl) using DNA Ligation Kit Ver.2 (Takara Shuzo Co., Ltd.). The resultant plasmid was introduced into a competent cell of E. coli, JM109, to give a transformant. The transformant was cultured overnight in 2xYT medium (2 mL) containing 50 µg/mL of ampicillin. From the cell fraction, the plasmid was isolated using QIAprep Spin Plasmid Kit (QIAGEN).

The plasmid obtained was sequenced for the cloned DNA portion. The sequencing was performed in 373A DNA Sequencer (ABI) using M13 Primer M4 and M13 Primer RV (Takara Shuzo Co., Ltd.). As a result, it was found that the cloned DNA had a 12-bp deletion therein. The plasmid was designated "CλΔ/pUC19". Then, for making up for the deleted portion, primers HCLMS (SEQ ID NO: 17) and HCLMR (SEQ ID NO: 18) were newly synthesized, and correct DNA was reconstructed using these primers by a PCR method.

In the first PCR, the plasmid CλΔ/pUC19 having the DNA deletion therein was used as a template, and the reaction was performed with each of the primer sets of HLAMBS and HCLMS and HCLMS and HLAMB4. The PCR products were purified separately. In the second PCR, the PCR products were assembled together. In the third PCR, the reaction product of the second PCR was added with external primers HLAMBS and HLAMB4 and amplified to give the full length DNA.

In the first PCR, a reaction solution (100 µl) containing 0.1 µg of CλΔ/pUC19 as a template, either 50 pmoles of each of the primers HLAMBS and HCLMR or 50 pmoles of each of the primers HCLMS and HLAMB4, and 5U of TaKaRa Ex Taq (Takara Shuzo Co., Ltd.) was used, over which 50 µl of mineral oil was layered. The PCR was run for 30 cycles under the conditions: 94°C for 1 min., 60°C for 1 min. and 72°C for 1 min.

The PCR products of the first PCR, HLAMBS-HCLMR (236 bp) and HCLMS-HLAMB4 (147 bp), were subjected to electrophoresis separately on a 3% low-melting agarose gel to isolate the DNA fragments. The DNA fragments were collected and purified from the gels using GENECLEAN II Kit (BIO101). In the second PCR, 20 µl of a reaction solution containing 40 ng of each of the purified DNA fragments and 1U of TaKaRa Ex Taq (Takara Shuzo Co., Ltd.) was used, over which 25 µl of mineral oil was layered. The PCR was run for 5 cycles under the conditions: 94°C for 1 min., 60°C for 1 min. and 72°C for 1 min.

In the third PCR, 100 µl of a reaction solution containing 2 µl of the reaction solution obtained by the second PCR, 50 pmoles of each of external primers HLAMBS and HLAMB4 and 5U of TaKaRa Ex Taq (Takara Shuzo Co., Ltd.) was used, over which 50 µl of mineral oil was layered. The PCR was run for 30 cycles under the conditions: 94°C for 1 min., 60°C for 1 min. and 72°C for 1 min., thereby obtaining a DNA fragment of 357 bp (the third PCR product). The DNA fragment was subjected to electrophoresis on a 3% low-melting agarose gel to isolate the DNA fragment. The resultant DNA fragment was collected and purified using GENECLEAN Kit (BIO101).

A portion (0.1 µg) of the DNA fragment thus obtained was digested with EcoRI, and then subcloned into plasmid pUC19 ΔHindIII that had been BAP-treated. The resultant plasmid was introduced into a competent cell of E. coli, JM109, to form a transformant. The transformant was cultured overnight in 2 mL of 2xYT medium containing 50 µg/mL of ampicillin. From the cell fraction, the plasmid was isolated and purified using QIAprep Spin Plasmid Kit (QIAGEN).

The purified plasmid was sequenced on 373A DNA Sequencer (ABI) using M13 Primer M4 and M13 Primer RV (Takara Shuzo Co., Ltd.). The plasmid that was confirmed to have the correct nucleotide sequence without any deletion was designated "Cλ/pUC19".

(iii) Construction of gene encoding human L-chain κ chain C-region

A DNA fragment encoding the L-chain κ chain C-region was cloned from plasmid HEF-PM1k-gk (WO 92/19759) by a PCR method. A forward primer HKAPS (SEQ ID NO: 19) was designed to contain EcoRI-, HindIII and BlnI-recognition sequences, and a backward primer HKAPA (SEQ ID NO: 20) was designed to contain an EcoRI-recognition sequence.

A PCR was performed using 100 μl of a reaction solution containing 0.1 μg of plasmid HEF-PM1k-gk as a template, 50 pmoles of each of primers HKAPS and HKAPA and 5U of TaKaRa Ex Taq (Takara Shuzo Co., Ltd.), over which 50 μl of mineral oil was layered. The PCR was run for 30 cycles under the conditions: 94°C for 1 min., 60°C for 1 min. and 72°C for 1 min., thereby giving a PCR product of 360 bp. The DNA fragment was isolated and purified by electrophoresis on a 3% low-melting agarose, and then collected and purified using GENECLEAN II Kit (BIO101).

The DNA fragment thus obtained was digested with EcoRI, and then cloned into plasmid pUC19 ΔHindIII that had been BAP-treated. The resultant plasmid was introduced into a competent cell of *E. coli,* JM109, to form a transformant. The transformant was cultured overnight in 2 mL of 2xYT medium containing 50 μg/mL of ampicillin. From the cell fraction, the plasmid was purified using QIAprep Spin Plasmid Kit (QIAGEN).

The purified plasmid was sequenced on 373A DNA Sequencer (ABI) using M13 Primer M4 and M13 Primer RV (Takara Shuzo Co., Ltd.). The plasmid that was confirmed to have the correct nucleotide sequence was designated "Cκ/pUC19".

(3) Construction of chimeric antibody L-chain expression vector

An expression vector for the chimeric #23-57-137-1 antibody L-chain was constructed. A gene encoding #23-57-137-1 L-chain V-region was ligated to the HindIII-BlnI site (located at the immediately front of the human antibody C-region) of each of the plasmids Cλ/pUC19 and Cκ/pUC19, thereby obtaining pUC19 vectors containing the DNA encoding the chimeric #23-57-137-1 antibody L-chain V-region and either the L-chain λ chain C-region or the L-chain κ region C-region. Each of the resultant vectors was then digested with EcoRI to separate the gene for the chimeric antibody L-chain. The gene was subcloned into HEF expression vector.

That is, a DNA fragment encoding #23-57-137-1 antibody L-chain V-region was cloned from plasmid MBC1L24 by a PCR method. Primers used in the PCR method were separately synthesized using 394 DNA/RNA Synthesizer (ABI). A backward primer MBCCHL1 (SEQ ID NO: 21) was designed to contain a HindIII-recognition sequence and a Kozak sequence (Kozak, M. et al., J. Mol. Biol. 196, 947-950, 1987), and a forward primer MBCCHL3 (SEQ ID NO: 22) was designed to contain BgIII- and RcoRI-recognition sequences.

PCR was performed using 100 μl of a reaction solution containing 10 mM Tris-HCl (pH 8.3), 50 mM KCl, 1.5 mM MgCl$_2$, 0.2 mM dNTPs, 0.1 μg MBC1L24, 50 pmoles of each of primers MBCCHL1 and MBCCHL3 and 1 μl of AmpliTaq (PERKIN ELMER), over which 50 μl of mineral oil was layered. The PCR was run for 30 cycles under the conditions: 94°C for 45 sec., 60°C for 45 sec. and 72°C for 2 min.

A PCR product of 444 bp was electrophoresed on a 3% low-melting agarose gel, and collected and purified using GENECLEAN II Kit (BIO101). The purified PCR product was dissolved in 20 μl of a solution containing 10 mM Tris-HCl (pH 7.4) and 1 mM EDTA. The PCR product (1 μl) was digested in 20 μl of a reaction solution containing 10 mM Tris-HCl (pH 7.5), 10 mM MgCl$_2$, 1 mM DTT, 50 mM NaCl, 8U of HindIII (Takara Shuzo Co., Ltd.) and 8U of EcoRI (Takara Shuzo Co., Ltd.) at 37°C for 1 hour. The digestion solution was subjected to phenol/chloroform extraction, and the DNA of interest was collected therefrom by ethanol precipitation. The DNA was dissolved in 8 μl of a solution containing 10 mM Tris-HCl (pH 7.4) and 1 mM EDTA.

In the same manner, plasmid pUC19 (1 μg) was digested with HindIII and EcoRI, and subjected to phenol/chloroform extraction and then ethanol precipitation. The obtained digested plasmid was BAP-treated with alkaline phosphatase (*E. coli* C75; Takara Shuzo Co., Ltd.). The resultant reaction solution was extracted with phenol and chloroform, and the DNA was collected therefrom by ethanol precipitation. The DNA was dissolved in 10 μl of a solution containing 10 mM Tris-HCl (pH 7.4) and 1 mM EDTA.

The BAP-treated plasmid pUC19 (1 μl) was ligated to the above-obtained PCR product (4 μl) using DNA Ligation Kit Ver. 2 (Takara Shuzo Co., Ltd.). The resultant plasmid was introduced into a competent cell of *E. coli,* JM109 (Nippon Gene Co., Ltd.) in the same manner as mentioned above, to form a transformant. The transformant was plated on 2xYT agar medium containing 50 μg/mL of ampicillin and cultured at 37°C overnight. The resultant transformant was then cultured at 37°C overnight in 2 mL of 2xYT medium containing 50 μg/mL of ampicillin. From the cell fraction, the plasmid was purified using QIAprep Spin Plasmid Kit (QIAGEN). After determining the nucleotide sequence, the plasmid that was confirmed to have the correct nucleotide sequence was designated "CHL/pUC19".

Each of plasmids Cλ/pUC19 and Cκ/pUC19 (1 μg each) was digested in 20 μl of a reaction solution containing 20 mM Tris-HCl (pH 8.5), 10 mM MgCl$_2$, 1 mM DTT, 100 mM KCl, 8U of HindIII (Takara Shuzo Co., Ltd.) and 2U of BlnI (Takara Shuzo Co., Ltd.) at 37°C for 1 hour. The digestion solution was extracted with phenol and chloroform, and the DNA was collected therefrom by ethanol precipitation. The DNA was BAP-treated at 37°C for 30 min. The reaction solution was extracted with phenol and chloroform, and the DNA was collected therefrom by ethanol precipitation. The DNA was dissolved in 10 μl of a solution containing 10 mM Tris-HCl (pH 7.4) and 1 mM EDTA.

The plasmid CHL/pUC19 that contained DNA encoding #23-57-137-1 L-chain V-region (8 μg) was digested with HindIII and BlnI in the same manner as mentioned above to give a DNA fragment of 409 bp. The DNA fragment was electrophoresed on a 3% low-melting agarose gel, and then collected and purified using GENECLEAN II Kit (BIO101) from the gel. The DNA was dissolved in 10 μl of a solution containing 10 mM Tris-HCl (pH 7.4) and 1 mM EDTA.

The DNA for the L-chain V-region (4 μl) was subcloned into 1 μl of each of the BAP-treated plasmids Cλ/pUC19 and Cκ/pUC19, and then introduced into a competent cell of *E. coli,* JM109, to form a transformant. The transformant was cultured overnight in 3 mL of 2xYT medium containing 50 μg/mL of ampicillin. From the cell fraction, the plasmid was isolated and purified using QIAprep Spin Plasmid Kit (QIAGEN). The two plasmids thus prepared were designated "MBC1L(λ)/pUC19" and "MBC1L(κ)/pUC19", respectively.

Each of plasmids MBC1L(λ)/pUC19 and MBC1L(κ)/pUC19 was digested with EcoRI and then subjected to electrophoresis on a 3% low-melting agarose gel. A DNA fragment of 743 bp was isolated and purified from the gel using GENECLEANII Kit (BIO101), and then dissolved in 10 μl of a solution containing 10 mM Tris-HCl (pH 7.4) and 1 mM EDTA.

An expression vector (plasmid HEF-PM1k-gk) (2.7 μg) was digested with EcoRI and then extracted with phenol and chloroform, and the DNA was collected therefrom by ethanol precipitation. The DNA fragment was BAP-treated, and then subjected to electrophoresis on a 1 % low-melting agarose gel. From the gel, a DNA fragment of 6561 bp was isolated and purified therefrom using GENECLEANII Kit (BIO101). The purified DNA fragment was dissolved in 10 μl of a solution containing 10 mM Tris-HCl (pH 7.4) and 1 mM EDTA.

The BAP-treated HEF vector (2 μl) was ligated to an EcoRI fragment (3 μl) of each of plasmid MBC1L(λ)/pUC19 and MBC1L(κ)/pUC19. The ligation product was introduced into a competent cell of *E. coli,* JM109, to form a transformant. The transformant was cultured in 2 mL of 2xYT medium containing 50 μg/mL of ampicillin. From the cell fraction, the plasmid was purified using QIAprep Spin Plasmid Kit (QIAGEN).

The purified plasmid was digested in 20 μl of a reaction solution containing 20 mM Tris-HCl (pH 8.5), 10mM MgCl$_2$, 1 mM DTT, 100 mM KCl, 8U of HindIII (Takara Shuzo Co., Ltd.) and 2 U of PvuI (Takara Shuzo Co., Ltd.) at 37°C for 1 hour. This reaction gave digestion fragments of 5104/2195 bp if the fragment was inserted in the correct orientation, or gave digestion fragments of 4378/2926 bp if the fragment was inserted in the reverse orientation. The plasmid that was confirmed to have the fragment in the correct orientation was designated "MBC1L(λ)/neo" for plasmid MBC1L(λ)/pUC19 or "MBC1L(κ)/neo" for plasmid MBC1L(κ)/pUC19.

(4) Transfection of COS-7 cell

To evaluate the antigen-binding activity and the neutralizing activity of the chimeric antibodies, the expression plasmids prepared above were separately expressed transiently in a COS-7 cell.

The transient expression of the chimeric antibodies was performed using each of the combinations of plasmids MBC1HcDNA/pCOS1 and MBC1L (λ)/neo and plasmids MBC1HcDNA/pCOS1 and MBC1L(κ)/neo, by co-tansfecting a COS-7 cell with the plasmids by electroporation using Gene Pulser (Bio Rad). That is, the plasmids (10 μg each) were added to a COS-7 cell suspension (0.8 mL; 1 x 10$^7$ cells/mL) in PBS(-). The resultant solution was applied with pulses at an electrostatic capacity of 1,500V and 2 μF to cause electroporation. After 10 min. of recovery period at room temperature, the electroporated cells were suspended in DMEM medium (GIBCO) containing 2% Ultra Low IgG fetal calf serum (GIBCO), and then cultured using a 10-cm culture dish in a CO$_2$ incubator. After cultivating for 72 hours, a culture supernatant was collected and centrifuged to remove cell debris and was provided for use as a sample for the subsequent ELISA. In this procedure, the purification of the chimeric antibody from the COS-7 cell culture supernatant was performed using AffiGel Protein A MAPSII Kit (Bio Rad) in accordance with the instructions included in the kit.

(5) ELISA

(i) Determination of antibody concentration

An ELISA plate for determining antibody concentration was prepared as follows. Each well of a 96-well ELISA plate (Maxisorp, NUNC) was coated with 100 μl of a coating buffer (0.1 M NaHCO$_3$, 0.02% NaN$_3$) supplemented

with 1 µg/mL of goat anti-human IgG antibody (TAGO), and then blocked with 200 µl of a dilution buffer [50 mM Tris-HCl, 1 mM $MgCl_2$, 0.1 M NaCl, 0.05% Tween 20, 0.02% $NaN_3$, 1% bovine serum albumin (BSA); pH 7.2]. Each well of the plate was added with each of the serial dilutions of the COS-7 cell culture supernatant in which each of the chimeric antibodies had been expressed, or added with each of the serial dilutions of each of the chimeric antibodies *per se* in a purified form. The plate was incubated at room temperature for 1 hour and washed with PBS-Tween 20. Each well of the plate was then added with 100 µl of a solution of alkaline phosphatase-conjugated goat anti-human IgG antibodies (TAGO). After the plate was incubated at room temperature for 1 hour and washed with PBS-Tween 20, each well was added with 1 mg/mL of a substrate solution ("Sigma 104", p-nitrophenylphosphoric acid, SIGMA). The solution was measured on its absorbance at 405 nm using Microplate Reader (Bio Rad) to determine the antibody concentration. In this determination, Hu IgG1λ Purified (The Binding Site) was used as the standard.

(ii) Determination of antigen-binding ability

An ELISA plate for the determination of antigen-binding ability was prepared as follows. Each well of a 96-well ELISA plate was coated with 100 µl of a coating buffer supplemented with 1 µg/mL of human PTHrP (1-34) (Peptide Research Institute), and then blocked with 200 µl of a dilution buffer. Each well was added with each of the serial dilutions of the COS-7 cell culture supernatant in which each of the chimeric antibodies had been expressed, or added with each of the serial dilutions of each of the chimeric antibodies *per se* in a purified form. After the plate was incubated at room temperature and washed with PBS-Tween 20, each well of the plate was added with 100 µl of a solution of alkaline phosphatase-conjugated goat anti-human IgG antibodies (TAGO). After the plate was incubated at room temperature and washed with PBS-Tween 20, each well of the plate was added with 1 mg/mL of a substrate solution ("Sigma 104", p-nitropbenylphosphoric acid, SIGMA). The solution was measured on its absorbance at 405 nm using Microplate Reader (Bio Rad).

As a result, it was found that the chimeric antibodies had an ability to bind to human PTHrP (1-34) and the cloned mouse antibody V-regions had the correct structures. It was also found that there was no difference in the ability to bind to PTHrP (1-34) between the chimeric antibody with L-chain λ chain C-region and the chimeric antibody with L-chain κ chain C-region. Therefore, the L-chain C-region of the humanized antibody was constructed using the humanized antibody L-chain λ chain.

(6) Establishment of CHO cell line capable of stable production of chimeric antibodies

To establish a cell line capable of producing the chimeric antibodies stably, the above-prepared expression plasmids were introduced into CHO cells (DXB11).

For the establishment of a cell line capable of producing the chimeric antibodies stably, either of the following combinations of the expression plasmids for CHO cell was used: MBC1HcDNA/pCHO1 and MBC1L(λ)/neo; and MBC1HcDNA/pCHO1 and MBC1L(κ)/neo. A CHO cell was co-transfected with the plasmids by electroporation using Gene Pulser (Bio Rad) as follows. The expression vectors were separately cleaved with a restriction enzyme PvuI to give linear DNAs. The resultant DNAs were extracted with phenol and chloroform and collected by precipitation with ethanol. The plasmid DNAs thus prepared were subjected to electroporation. That is, each of the plasmid DNAs (10 µg each) was added to 0.8 mL of a cell suspension of CHO cells in PBS(-) ($1 \times 10^7$ cells/mL). The resultant solution was applied with pulses at an electrostatic capacity of 1,500V and 25 µF. After 10 min. of recovery period at room temperature, the electroporated cells were suspended in MEM-α medium (GIBCO) containing 10% fetal calf serum (GIBCO). The resultant suspension was cultured using three 96-well plates (Falcon) in a $CO_2$ incubator. On the day after starting the cultivation, the medium was replaced by a selective medium [ribonucleoside- or deoxyribonucleoside-free MEM-α medium (GIBCO) containing 10% fetal calf serum (GIBCO) and 500 mg/mL of GENETICIN (G418Sulfate; GIBCO)]. From the culture medium, cells into which the antibody gene was introduced were selected. The selective medium is replaced by a fresh one. About two weeks after the medium replacement, the cells were observed under a microscope. When a favorable cell growth was observed, the cells were determined on the amount of the produced antibodies by ELISA as mentioned above. Among the cells, those which produced a larger amount of antibodies were screened.

Then, the cultivation of the established cell line capable of stable production of the antibodies was scaled up in a roller bottle using ribonucleoside- or deoxyribonucleoside-free MEM medium containing 2% Ultra Low IgG fetal calf serum. On day 3 and day 4 of the cultivation, the culture supernatant was collected and then filtered using a 0.2-µm filter (Millipore) to remove cell debris therefrom.

Purification of the chimeric antibodies from the CHO cell culture supernatant was performed using POROS Protein A Column (PerSeptive Biosystems) on ConSep LC100 (Millipore) in accordance with the instructions included in the kit. The purified chimeric antibodies were provided for use as samples for the determination of neutralizing activity and for the examination of therapeutic efficacy on hypercalcemic model animals. The concentration and the antigen-binding activity of the purified chimeric antibodies were determined using the same ELISA system as mentioned above.

[REFERENCE EXAMPLE 4] Construction of humanized antibody

(1) Construction of humanized antibody H-chain

(i) Construction of humanized H-chain V-region

A humanized #23-57-137-1 antibody H-chain was produced by CDR-grafting technique by means of a PCR method. For the production of a humanized #23-57-137-1 antibody H-chain (version "a") having FRs derived from human antibody S31679 (NBRF-PDB; Cuisinier, A. M. et al., Eur. J. Immunol., 23, 110-118, 1993), the following six PCR primers were used: CDR-grafting primers: MBC1HGP1 (SEQ ID NO: 23) and MBC1HGP3 (SEQ ID NO: 24) (both containing a sense DNA sequence) and MBC1HGP2 (SEQ ID NO: 25) and MBC1HGP4 (SEQ ID NO: 26) (both containing an antisense DNA sequence), all of which containing a 15-21 bp complementary sequence on both terminal ends thereof; and external primers: MBC1HVS1 (SEQ ID NO: 27) and MBC1HVR1 (SEQ ID NO: 28) having a homology to the CDR-grafting primers MBC1HGP1 and MBC1HGP4, respectively.

The CDR-grafting primers MBC1HGP1, MBC1HGP2, MBC1HGP3 and MBC1HGP4 were separated on an urea-denatured polyacrylamide gel (Molecular Cloning: A Laboratory Manual, Sambrook, et al., Cold Spring Harbor Laboratory Press, 1989), and extracted therefrom by a crush-and-soak method (Molecular Cloning: A Laboratory Manual, Sambrook, et al., Cold Spring Harbor Laboratory Press, 1989) in the following manner.

Each of the CDR-grafting primers (1 nmole) was separated on a 6% denatured polyacrylamide gel to give DNA fragments. From the resultant DNA fragments, one having a desired length was identified on a silica gel thin plate by irradiation of UV ray and then collected from the gel by a crush-and-soak method. The resultant DNA was dissolved in 20 μl of a solution containing 10 mM Tris-HCl (pH 7.4) and 1 mM EDTA. PCR was performed using TaKaRa Ex Taq (Takara Shuzo Co., Ltd.). The PCR reaction solution (100 μl) comprised 1 μl of each of the above-mentioned CDR-grafting primers MBC1HGP1, MBC1HGP2, MBC1HGP3 and MBC1HGP4, 0.25 mM dNTPs and 2.5U of TaKaRa Ex Taq in the buffer. The PCR was run for 5 cycles under the conditions: 94°C for 1 min., 55°C for 1 min. and 72°C for 1 min. The resultant reaction solution was added with the external primers MBC1HVS1 and MBC1HVR1 (50 pmoles each). Using this reaction mixture, the PCR was further run for additional 30 cycles under the same conditions. The DNA fragment thus amplified was separated by agarose gel electrophoresis on a 4% Nu Sieve GTG agarose (FMC Bio. Products).

An agarose segment containing a DNA fragment of 421 bp was excised, and the DNA fragment was purified therefrom using GENECLEANII Kit (BIO101) in accordance with the instructions included in the kit. The DNA fragment thus purified was precipitated with ethanol and then dissolved in 20 μl of a solution containing 10 mM Tris-HCl (pH 7.4) and 1 mM EDTA. The resultant PCR reaction mixture was used for subcloning of the DNA fragment into plasmid pUC19 that had been digested with BamHI and HindIII, and subsequently the nucleotide sequence of the resultant plasmid was determined. A plasmid having the correct nucleotide sequence was designated "hMBCHv/pUC19".

(ii) Construction of H-chain V-region for Humanized H-chain cDNA

To ligate to cDNA for humanized H-chain C-region Cγ1, the DNA for the humanized H-chain V-region constructed in the above step was modified by a PCR method. For the PCR method, a backward primer MBC1HVS2 was designed to hybridize to the sequence encoding the 5' region of the leader sequence for the V-region and to have a Kozak consensus sequence (Kozak M. et al., J. Mol. Biol. 196, 947-950, 1987) and HindIII- and EcoRI-recognition sequences; and a forward primer MBC1HVR2 was designed to hybridize to both the DNA sequence encoding the 3' region of the J region and the DNA sequence encoding the 5' region of the C-region and to have ApaI- and SmaI-recognition sequences.

PCR was performed using TaKaRa Ex Taq (Takara Shuzo Co., Ltd.) and a buffer appended thereto. The PCR reaction solution comprised 0.4 μg of hMBCHv/pUC19 as a DNA template, 50 pmoles of each of MBC1HVS2 and MBC1HVR2 as primers, 2.5U of TaKaRa Ex Taq and 0.25 mM dNTPs in the buffer. The PCR was run for 30 cycles under the conditions: 94°C for 1 min., 55°C for 1 min. and 72°C for 1 min. The DNA fragment thus amplified was separated by agarose gel electrophoresis on a 3% Nu Sieve GTG agarose (FMC Bio. Products).

A gel segment containing a DNA fragment of 456 bp was excised, and the DNA fragment was purified therefrom using GENECLEANII Kit (BIO101) in accordance with the instructions included in the kit. The DNA fragment thus purified was precipitated with ethanol and then dissolved in 20 μl of a solution containing 10 mM Tris-HCl (pH 7.4) and 1 mM EDTA. The PCR reaction solution thus obtained was used for subcloning of the DNA fragment into plasmid pUC19 that had been digested with EcoRI and SmaI, and then the resultant plasmid was sequenced. As a result, a plasmid was obtained which contained DNA encoding mouse H-chain V-region derived from hybridoma #23-57-137-1 and also contained EcoRI- and HindIII-recognition sequences and a Kozak sequence on the 5' region and ApaI- and SmaI-recognition sequences on the 3' region, which was designated "hMBC1Hv/pUC19".

(2) Construction of expression vector for humanized antibody H-chain

Plasmid RVh-PM1f-cDNA carrying a cDNA sequence for hPM1 antibody H-chain was digested with ApaI and BamHI to give a DNA fragment containing a DNA fragment containing DNA encoding the H-chain C-region. The DNA fragment was introduced into plasmid hMBC1Hv/pUC19 that had been digested with ApaI and BamHI. The obtained plasmid was designated "hMBC1HcDNA/pUC19". This plasmid contained both DNA encoding the humanized #23-57-137-1 antibody H-chain V-region and DNA encoding the human H-chain C-region C$\gamma$1 and had EcoRI-and HindIII-recognition sequences on the 5' region and a BamHI-recognition sequence on the 3' region. The nucleotide sequence and the corresponding amino acid sequence for the humanized H-chain version "a" carried on the plasmid hMBC1HcDNA/pUC19 are shown in SEQ ID NO: 58 and SEQ ID NO: 56, respectively.

The plasmid hMBC1HcDNA/pUC19 was digested with EcoRI and BamHI to give a DNA fragment containing DNA encoding the H-chain. The DNA fragment was introduced into expression plasmid pCOS1 that had been digested with EcoRI and BamHI. As a result, an expression plasmid for a humanized antibody was obtained, which was designated "hMBC1HcDNA/pCOS1".

To produce a plasmid used for expression in a CHO cell, plasmid hMBC1HcDNA/pUC19 was digested with EcoRI and BamHI to give a DNA fragment containing DNA encoding the H-chain. The DNA fragment was introduced into expression vector pCHO1 that had been digested with EcoRI and BamHI. As a result, an expression plasmid for the humanized antibody was obtained, which was designated "hMBC1HcDNA/pCHO1".

(3) Construction of L-chain hybrid V-region

(i) Preparation of FR1,2/FR3,4 hybrid antibody

A gene for the FR hybrid L-chain having both FRs from a humanized antibody and FRs from a mouse (chimeric) antibody was constructed, and each region was evaluated for the humanization. In this step, a hybrid antibody having FR1 and FR2 both derived from a human antibody and FR3 and FR4 both derived from a mouse antibody was prepared by utilizing the AflII restriction site located on CDR2.

Plasmids MBC1L($\lambda$)/neo and hMBC1L($\lambda$)/neo (10 $\mu$g each) were separately digested in 100 $\mu$l of a reaction solution containing 10 mM Tris-HCl (pH 7.5), 10 mM MgCl$_2$, 1 mM DTT, 50 mM NaCl, 0.01% (w/v) of BSA and 10 U of AflII (Takara Shuzo Co., Ltd.) at 37°C for 1 hour. The reaction solutions were subjected to electrophoresis on a 2% low-melting agarose gel, thereby giving DNA fragments of 6282 bp (referred to as "c1") and 1022 bp (referred to as "c2") from the plasmid MBC1L($\lambda$)/neo or DNA fragments of 6282 bp (referred to as "h1" ) and 1022 bp (referred to as "h2") from the plasmid hMBC1L($\lambda$)/neo. These DNA fragments were collected and purified from the gels using GENECLEANII Kit (BIO101).

Each of the c1 and h1 fragments (1 $\mu$g) was BAP-treated. The DNA fragment was extracted with phenol and chloroform, collected by ethanol precipitation, and dissolved in 10 $\mu$l of a solution containing 10 mM Tris-HCl (pH 7.4) and 1 mM EDTA.

The BAP-treated c1 and h1 DNA fragments (1 $\mu$l each) were ligated to the h2 and c2 DNA fragments (4 $\mu$l each), respectively, (at 4°C overnight). Each of the ligation products was introduced into a competent cell of *E. coli,* JM109, to form a transformant. The transformant was cultured in 2 mL of 2xYT medium containing 50 $\mu$g/mL of ampicillin. From the cell fraction, the plasmid was purified using QIAprep Spin Plasmid Kit (QIAGEN).

The purified plasmid was digested in 20 $\mu$l of a reaction solution containing 10 mM Tris-HCl (pH 7.5), 10 mM MgCl$_2$, 1 mM DTT, and either 2U of ApaLI (Takara Shuzo Co., Ltd.) or 8U of BamHI (Takara Shuzo Co., Ltd.) and HindIII (Takara Shuzo Co., Ltd.) at 37°C for 1 hour. If the c1-h2 was ligated correctly, this digestion reaction gave fragments of 5560/1246/498 bp (by the ApaLI digestion) or fragments of 7134/269 bp (by the BamHI/HindIII digestion). Based on this assumption, the desired plasmids were identified.

The expression vector encoding the human FR1,2/mouse FR3,4 hybrid antibody L-chain was designated "h/mMBC1L($\lambda$)/neo". On the other hand, a clone for the h1-c1 could not be obtained. Therefore, recombination on a pUC vector was performed, and then the resultant recombinant product was cloned into a HEF vector. In this procedure, plasmid hMBC1La$\lambda$/pUC19, which contained DNA encoding a humanized antibody L-chain V-region without any amino acid replacements, and plasmid hMBC1Ld$\lambda$/pUC19, which contained DNA encoding a humanized antibody L-chain V-region with an amino acid replacement at the 91-position amino acid tyrosine in FR3 (i.e., the 87th amino acid in accordance with The Kabat's prescription) by isoleucine, were used as templates.

Plasmids MBC1L($\lambda$)/pUC19, hMBC1La$\lambda$/pUC19 and hMBC1Ld$\lambda$/pUC19 (10 $\mu$g each) were separately digested in 30 $\mu$l of a reaction solution containing 10 mM Tris-HCl (pH 7.5), 10 mM MgCl$_2$, 1 mM DTT, 50 mM NaCl, 0.01% (w/v) of BSA, 16U of HindIII and 4U of AflII at 37°C for 1 hour. The reaction solutions were separately subjected to electrophoresis on a 2% low-melting agarose gel, thereby giving a DNA fragment 215 bp from plasmid MBC1L($\lambda$)/pUC19 (referred to as "c2'") and a DNA fragment of 3218bp from each of plasmids hMBC1La$\lambda$/pUC19 and

hMBC1Ldλ/pUC19 (referred to as "ha1'" and "hd1'", respectively). These DNA fragments were collected and purified using GENECLEANII Kit (BIO101).

Each of the ha1' and hd1' fragments was ligated to the c2' fragment and then introduced into a competent cell of *E. coli,* JM109, to form a transformant. The transformant was cultured in 2 mL of 2xYT medium containing 50 μg/mL of ampicillin. From the cell fraction, the plasmid was purified using QIAprep Spin Plasmid Kit (QIAGEN). The plasmids thus prepared were designated "m/hMBC1Laλ/pUC19" for the ha1' fragment-containing plasmid and "m/hMBC1Ldλ/pUC19" for the hd1' fragment-containing plasmid.

Each of the plasmids m/hMBC1Laλ/pUC19 and m/hMBC1Ldλ/pUC19 was digested with EcoRI. The DNA fragment of 743 bp was electrophoresed on a 2% low-melting agarose gel, and then collected and purified therefrom using GENECLEANII Kit (BIO101). The resultant DNA fragment was dissolved in 20 μl of a solution containing 10 mM Tris-HCl (pH 7.4) and 1 mM EDTA.

Each of the DNA fragments (4 μl each) was ligated to the above-obtained BAP-treated HEF vector (1 μl). The ligation product was introduced into a competent cell of *E. coli,* JM109, to form a transformant. The transformant was cultured in 2 mL of 2xYT medium containing 50 μg/mL of ampicillin. From the cell fraction, the plasmid was purified using QIAprep Spin Plasmid Kit (QIAGEN).

Each of the purified plasmids was digested in 20 μl of a reaction solution containing 20 mM Tris-HCl (pH 8.5), 10 mM MgCl$_2$, 1 mM DTT, 100 mM KCl, 8U of HindIII (Takara Shuzo Co., Ltd.) and 2U of PvuI (Takara Shuzo Co., Ltd.) at 37°C for 1 hour. The plasmid DNA was identified based on the expectation that: if the DNA fragment was inserted in the plasmid in a correct orientation, this digestion would give a digestion fragment of 5104/2195 bp, whereas if the DNA fragment is inserted in the plasmid in the reverse orientation, this digestion would give a digestion fragment of 4378/2926 bp. The plasmids thus obtained were expression vectors coding for mouse FR1,2/human FR3,4 hybrid antibody L-chain, which were designated expression vectors "m/hMBC1Laλ/neo" and "m/hMBC1Ldλ/ neo", respectively.

(ii) Preparation of FR1/FR2 hybrid antibody

An FR1/FR2 hybrid antibody was prepared in the same manner as mentioned above utilizing a SnaBI restriction site located on CDR1.

Plasmids MBC1L(λ)/neo and h/mMBC1L(λ)/neo (10 μg each) were separately digested in 20 μl of a reaction solution containing 10 mM Tris-HCl (pH 7.9), 10 mM MgCl$_2$, 1 mM DTT, 50 mM NaCl, 0.01% (w/v) of BSA and 6U of SnaBI (Takara Shuzo Co., Ltd.) at 37°C for 1 hour. The resultant reaction solutions were further digested in 50 μl of a reaction solution containing 20 mM Tris-HCl (pH 8.5), 10 mM MgCl$_2$, 1 mM DTT, 100 mM KCl, 0.01% (w/v) of BSA and 6U of PvuI at 37°C for 1 hour.

The resultant reaction solutions were separately subjected to electrophoresis on a 1.5% low-melting agarose gel, thereby giving DNA fragments of 4955 bp (m1) and 2349 bp (m2) from the plasmid MBC1L(λ)/neo and DNA fragments of 4955 bp (hm1) and 2349 bp (hm2) from the plasmid h/mMBC1L(λ)/neo. These DNA fragments were collected and purified from the gels using GENECLEANII Kit (BIO101). Each of the DNA fragments obtained was dissolved in 40 μl of a solution containing 10 mM Tris-HCl (pH 7.4) and 1 mM EDTA.

The m1 and hm1 fragments (1 μl each) were ligated to the hm2 and m2 fragments (4 μl each), respectively. Each of the resultant ligation products was introduced into a competent cell of *E. coli,* JM109, to form a transformant. The transformant obtained was cultured in 2 mL of 2xYT medium containing 50 μg/mL of ampicillin. From the cell fraction, the plasmid was purified using QIAprep Spin Plasmid Kit QIAGEN).

Each of the purified plasmids was digested in 20 μl of a reaction solution containing 10 mM Tris-HCl (pH 7.5), 10 mM MgCl$_2$, 1 mM DTT and either 8U of ApaI (Takara Shuzo Co., Ltd.) or 2U of ApaLI (Takara Shuzo Co., Ltd.) at 37°C for 1 hour.

If the fragments were ligated correctly, the digestion reaction would give a fragment of 7304 bp (by the ApaI digestion) or fragments of 5560/1246/498 bp (by the ApaLI digestion) for m1-hm2, and fragments of 6538/766 bp (by the ApaI digestion) or fragments of 3535/2025/1246/498 bp (by the ApaLI digestion) for hm1-m2. Based on this assumption, the plasmids were identified. As a result, an expression vector encoding a human FR1/mouse FR2,3,4 hybrid antibody L-chain (designated "hmmMBC1L(λ)/neo") and an expression vector encoding a mouse FR1/human FR2/mouse FR3,4 hybrid antibody L-chain (designated "mhmMBC1L(X)/neo") were obtained.

(4) Construction of humanized antibody L-chain

A humanized #23-57-137-1 antibody L-chain was prepared by CDR-grafting technique by means of PCR method. For the preparation of a humanized #23-57-137-1 antibody L-chain (version "a") that contained FR1, FR2 and FR3 derived from human antibody HSU03868 (GEN-BANK, Deftos M. et al., Scand. J. Immunol., 39, 95-103, 1994) and FR4 derived from human antibody S25755 (NBRF-PDB), six PCR primers were used.

The six primers were as follows: CDR-grafting primers MBC1LGP1 (SEQ ID NO: 29) and MBC1LGP3 (SEQ ID NO: 30), both having a sense DNA sequence, CDR-grafting primers MBC1LGP2 (SEQ ID NO: 31) and MBC1LGP4 (SEQ ID NO: 32), both having an antisense DNA sequence, all of which had a 15-21 bp complementary sequence on the both terminal ends; and external primers MBC1LVS1 (SEQ ID NO: 33) and MBC1LVR1 (SEQ ID NO: 34) having a homology to the CDR-grafting primers MBC1LGP1 and MBC1LGP4, respectively.

The CDR-grafting primers MBC1LGP1, MBC1LGP2, MBC1LGP3 and MBC1LGP4 were separated on a urea-denatured polyacrylamide gel (Molecular Cloning: A Laboratory Manual, Sambrook et al., Cold Spring Harbor Laboratory Press, 1989) and extracted therefrom segment by a crush-and-soak method (Molecular Cloning: A Laboratory Manual, Sambrook et al., Cold Spring Harbor Laboratory Press, 1989).

Each of the CDR-grafting primers (1 nmole) was separated with 6% denatured polyacrylamide gel. The identification of the DNA fragment of a desired length was performed on a silica gel thin plate by irradiation of UV ray. The desired DNA fragment was collected from the gel by a crush-and-soak method. The collected DNA fragment was dissolved in 20 μl of a solution containing 10 mM Tris-HCl (pH 7.4) and 1 mM EDTA.

PCR was performed using TaKaRa Ex Taq (Takara Shuzo Co., Ltd.) and a buffer appended thereto. The PCR reaction solution comprised (per 100 μl) 1 μl of each of the CDR-grafting primers MBC1LGP1, MBC1LGP2, MBC1LGP3 and MBC1LGP4, 0.25 mM dNTPs, 2.5U of TaKaRa Ex Taq in the buffer. The PCR was run for 5 cycles under the conditions: 94°C for 1 min., 55°C for 1 min. and 72°C for 1 min. The resultant reaction mixture was added with 50 pmoles of each of the external primers MBC1LVS1 and MBC1LVR1. Using this reaction mixture, the PCR was run for additional 30 cycles under the same conditions. The DNA fragment thus amplified was separated by agarose gel electrophoresis on a 3% Nu Sieve GTG agarose (FMC Bio. Products).

An agarose segment containing a DNA fragment of 421 bp was excised, and the DNA fragment was purified therefrom using GENECLEANII Kit (BIO101) in accordance with the instructions included in the kit. The PCR reaction mixture thus obtained was used for subcloning of the DNA fragment into plasmid pUC19 that had been digested with BamHI and HindIII. The resultant plasmid was sequenced. The plasmid thus prepared was designated "hMBCL/pUC19". In this plasmid, however, the 104-position amino acid (corresponding to the 96th amino acid in accordance with the Kabat's prescription) of CDR4 was replaced by arginine. For the correction of this amino acid to tyrosine, a correction primer MBC1LGP10R (SEQ ID NO: 35) was designed and synthesized. PCR was performed using TaKaRa Ex Taq (Takara Shuzo Co., Ltd.) and a buffer appended thereto. The PCR reaction solution comprised (per 100 μl) 0.6 μg of the plasmid hMBCL/pUC19 as a template DNA, 50 pmoles of each of the primers MBC1LVS1 and MBC1LGP10R, 2.5U of TaKaRa Ex Taq (Takara Shuzo Co., Ltd.) and 0.25 mM dNTPs in the buffer, over which mineral oil (50 μl) was layered. The PCR was run for 30 cycles under the conditions: 94°C for 1 min., 55°C for 1 min. and 72°C for 1 min. The DNA fragment thus amplified was separated by agarose gel electrophoresis on a 3% Nu Sieve GTG agarose (FMC Bio. Products).

A gel segment containing a DNA fragment of 421 bp was excised, and the DNA fragment was purified therefrom using GENECLEANII Kit (BIO101) in accordance with the instructions included in the kit. The PCR reaction mixture thus prepared was used for subcloning of the DNA fragment into plasmid pUC19 that had been digested with BamHI and HindIII.

The plasmid was sequenced using M13 Primer M4 and M13 Primer RV. As a result, it was confirmed that the plasmid had the correct sequence. The plasmid was then digested with HindIII and BlnI, and a DNA fragment of 416 bp was separated by electrophoresis on a 1% agarose gel. The DNA fragment was purified using GENECLEANII Kit (BIO101) in accordance with the instructions included in the kit, and then introduced into plasmid Cλ/pUC19 that had been digested with HindIII and BlnI. The resultant plasmid was designated "hMBC1Laλ/pUC19". This plasmid was digested with EcoRI to give a DNA fragment encoding humanized L-chain. The DNA fragment was introduced into plasmid pCOS1 so that the initiation codon for the humanized L-chain was located downstream to the EF1α promoter. The plasmid thus obtained was designated "hMBC1Laλ/pCOS1". The DNA sequence (including the corresponding amino acid sequence) of the humanized L-chain version "a" is shown in SEQ ID NO: 66. The amino acid sequence of the version "a" is shown in SEQ ID NO: 47.

A humanized L-chain version "b" was prepared using a mutagenesis technique by a PCR method. The version "b" was designed such that the 43-position amino acid glycine (corresponding to the 43th amino acid in accordance with the Kabat's prescription) was replaced by proline and the 49-position amino acid lysine (corresponding to the 49th amino acid in accordance with the Kabat's prescription) by aspartic acid in the version "a". PCR was performed using plasmid hMBC1Laλ/pUC19 as a template with a mutagenic primer MBC1LGP5R (SEQ ID NO: 36) and a primer MBC1LVS1. The DNA fragment obtained was digested with BamHI and HindIII, and the digestion fragment was subcloned into the BamHI-HindIII site of pUC19. After sequencing, the plasmid was digested with HindIII and AflII, and the resultant digestion fragment was ligated to plasmid hMBC1Laλ/pUC19 that had been digested with HindIII and AflII.

The plasmid thus obtained was designated "hMBC1Lbλ/pUC19". This plasmid was digested with EcoRI to give a DNA fragment containing DNA encoding the humanized L-chain. The DNA fragment was introduced into plasmid pCOS1 such that the initiation codon for the humanized L-chain was located downstream to the EF1α promoter. The plasmid thus obtained was designated "hMBC1Lbγ/pCOS1".

A humanized L-chain version "c" was prepared using a mutagenesis technique by a PCR method. The version "c" was designed such that the 84-position amino acid serine (corresponding to the 80th amino acid in accordance with the Kabat's prescription) was replaced by proline. PCR was performed using plasmid hMBC1Laλ/pUC19 as a template with a mutagenic primer MBC1LGP6S (SEQ ID NO: 37) and a primer M13 Primer RV. The DNA fragment obtained was digested with BamHI and HindIII and then subcloned into pUC19 that had been digested with BamHI and HindIII.

After sequencing, the plasmid was digested with BstPI and Aor51HI, and the resultant DNA fragment was ligated to plasmid hMBC1Laλ/pUC19 that had been digested with BstPI and Aor51HL The plasmid thus obtained was designated "hMBC1Lcλ/pUC19". This plasmid was digested with EcoRI to give a DNA fragment containing DNA encoding the humanized L-chain. The fragment was introduced into the EcoRI site of plasmid pCOS1 such that the initiation codon for the humanized L-chain was located downstream to the EF1α promoter. The plasmid thus obtained was designated "hMBC1Lcλ/pCOS1".

Humanized L-chain versions "d", "e" and "f" were also prepared using a mutagenesis technique by a PCR method. The versions "d", "e" and "f" were designed such that the 91-position amino acid tyrosine (corresponding to the 87th amino acid in accordance with the Kabat's prescription) was replaced by isoleucine in the versions "a", "b" and "c", respectively. For each of the versions "d", "e" and "f", a PCR was performed using each of plasmid hMBC1Laλ/pCOS1 (for version "d"), hMBC1Lbλ/pCOS1 (for version "e") and hMBC1Lcλ/pCOSI (for version "f"), respectively, as a template, a mutagenic primer MBC1LGP11R (SEQ ID NO: 38) and a primer M-S1 (SEQ ID NO: 44). The DNA fragment thus obtained was digested with BamHI and HindIII and then subcloned into pUC19 that had been digested with BamHI and HindIII. After sequencing, the plasmid was digested with HindIII and BlnI, and the resultant digestion fragment was ligated to plasmid Cλ/pUC19 that had been digested with HindIII and BlnI.

The plasmids thus obtained were respectively designated "hMBC1Ldλ/pUC19" (for version "d"), "hMBC1Leλ/pUC19" (for version "e") and "hMBC1Lfλ/pUC19" (for version "f"). Each of these plasmids was digested with EcoRI to give a DNA fragment containing DNA encoding the humanized L-chain. The DNA fragment was introduced into the EcoRI site of plasmid pCOS1 such that the initiation codon for the humanized L-chain was located downstream to the EF1α promoter of the plasmid. The plasmids thus obtained were respectively designated "hMBC1Ldλ/pCOS1" (for version "e"), "hMBC1Leλ/pCOS1" (for version "e") and "hMBC1Lfλ/pCOS1" (for version "f").

Humanized L-chain versions "g" and "h" were also prepared using a mutagenesis technique by a PCR method. The versions "g" and "h" were designed such that the 36-position amino acid histidine (corresponding to the 36th amino acid in accordance with the Kabat's prescription) was replaced by tyrosine in the versions "a" and "d", respectively. PCR was performed using a mutagenic primer MBC1LGP9R (SEQ ID NO: 39), M13 Primer RV and plasmid hMBC1Laλ/pUC19 as a template. An additional PCR was performed using the PCR product thus obtained and M13 Primer M4 as a primer and plasmid hMBC1Laλ/pUC19 as a template. The DNA fragment obtained was digested with HindIII and BlnI and then subcloned into plasmid Cλ/pUC19 that had been digested with HindIII and BlnI. Using this plasmid as a template, a PCR was performed with primers MBC1LGP13R (SEQ ID NO: 40) and MBC1LVS1. The PCR fragment obtained was digested with ApaI and HindIII and then introduced into either plasmids hMBC1Laλ/pUC19 or hMBC1Ldλ/pUC19 that had been digested with ApaI and HindIII. The plasmids obtained were sequenced. Plasmids that were confirmed to contain the correct sequence were designated "hMBC1Lgλ/pUC19" (for version "g") and "hMBC1Lhλ/pUC19" (for version "h"). Each of these plasmids was digested with EcoRI to give a DNA fragment containing DNA encoding the humanized L-chain. The DNA fragment was introduced into the EcoRI site of plasmid pCOS1 such that the initiation codon for the humanized L-chain was located downstream to the EF1α promoter. The plasmids thus obtained were respectively designated "hMBC1Lgλ/pCOS1" (for version "g") and "hMBC1Lhλ/pCOS1" (for version "h").

Humanized L-chain versions "i", "j", "k", "l", "m", "n" and "o" were also prepared using a mutagenesis technique by a PCR method. PCR was performed using plasmid hMBC1Laλ/pUC19 as a template with a mutagenic primer MBC1LGP14S (SEQ ID NO: 41) and a primer V1RV (λ) (SEQ ID NO: 43). The resultant DNA fragment was digested with ApaI and BlnI and then subcloned into plasmid hMBC1Lgλ/pUC19 that had been digested with ApaI and BlnI. The plasmid obtained was sequenced, and the clone into which the mutation for each version was introduced was selected. The plasmid thus obtained was designated "hMBC1Lxλ/pUC19 (x=i, j, k, l, m, n or o)". This plasmid was digested with EcoRI to give a DNA fragment containing DNA encoding the humanized L-chain. The DNA fragment was introduced into the EcoRI site of plasmid pCOS1 such that the initiation codon for the humanized L-chain was located downstream to the EF1α promoter. The plasmid thus obtained was designated "hMBC1Lxλ/pCOS1" (x = i, j, k, l, m, n or o). The DNA sequences (including the corresponding amino acid sequences) of the versions "j", "l", "m" and "o" are shown in SEQ ID NOs: 67, 68, 69 and 70, respectively. The amino acid sequences of these versions are also shown in SEQ ID Nos: 48, 49, 50 and 51, respectively.

Humanized L-chain versions "p", "q", "r", "s" and "t" were designed such that the 87-position amino acid (tyrosine) was replaced by isoleucine in the versions "i", "j", "m", "l" and "o", respectively. These versions were prepared utilizing the Aor51MI restriction site of FR3 and replacing that site of each of the versions "i", "j", "m", "l" or "o" by that site of the version "h". That is, an Aor51HI restriction fragment (514 bp) containing CDR3, a portion of FR3 and the entire FR4 were removed from an expression plasmid hMBC1Lxλ/pCOS1 (x = i, j, m, l or o). To the removed site, an Aor51HI restriction fragment (514 bp) in the expression plasmid hMBC1Lhλ/pCOS, which containing CDR3 and a portion of FR3 and the entire FR4, was ligated, so that the 91-position amino acid tyrosine (corresponding to the 87th amino acid in accordance with the Kabat's prescription) was replaced by isoleucine. The resultant plasmid was sequenced. A clone of each of the versions "i", "j", "m" "l" and "o" in which 91-position amino acid tyrosine (corresponding to the 87th amino acid in accordance with the Kabat's prescription) was replaced by isoleucine was selected. These modified versions respectively corresponding to the versions "i", "j", "m" "l" and "o" were designated versions "p", "q", "s", "r" and "t", respectively. The obtained plasmid was designated "hMBC1Lxλ/pCOS1 (x =p, q, s, r or t). The DNA sequences (including the corresponding amino acids) of the versions "q", "r" "s" and "t" are shown in SEQ ID Nos: 71, 72, 73 and 74, respectively. The amino acid sequences of these versions are also shown in SEQ ID Nos: 52, 53, 54 and 55, respectively.

Plasmid hMBC1Lqλ/pCOS1 was digested with HindIII and EcoRI and then subcloned into plasmid pUC19 that had been digested with HindIII and EcoRI. The plasmid thus obtained was designated "hMBC1Lqλ/pUC19.

The positions of the replaced amino acids in the individual versions of the humanized L-chain are shown in Table 2.

Table 2

| Positions of replaced amino acid in sequence listings (amino acid numbering in accordance with the Kabat's prescription) | | | | | | | |
|---|---|---|---|---|---|---|---|
| Versions | 36 | 43 | 45 | 47 | 49 | 80 | 87 |
| a | | | | | | | |
| b | | P | | | D | | |
| c | | | | | | P | |
| d | | | | | | | I |
| e | | P | | | D | | I |
| f | | | | | | P | I |
| g | Y | | | | | | |
| h | Y | | | | | | I |
| i | Y | | K | | | | |
| j | Y | | K | | D | | |
| k | Y | | K | V | | | |
| l | Y | | K | V | D | | |
| m | Y | | | | D | | |
| n | Y | | | V | | | |
| o | Y | | | V | D | | |
| p | Y | | K | | | | I |
| q | Y | | K | | D | | I |
| r | Y | | | | D | | I |
| s | Y | | K | V | D | | I |
| t | Y | | | V | D | | I |
| In Table 2, capital letters represent the following amino acids: Y: tyrosine; P: proline; K: lysine, V: valine; D: aspartic acid; and I: isoleucine. | | | | | | | |

E. coli strains each containing plasmids hMBC1HcDNA/pUC19 and hMBC1Lqλ/pUC19 were designated "Escherichia coli JM109 (hMBC1HcDNA/pUC19)" and "Escherichia coli JM109 (hMBC1Lqλ/pUC19)", respectively, which have been deposited under the terms of Budapest Treaty at the National Institute of Bioscience and Human-Technology, Agency of Industrial Science and Technology, Japan, (1-3, Higashi 1-chome, Tsukuba-shi, Ibaraki, Japan) on August 15, 1996, under the accession No. FERM BP-5629 for *Escherichia coli* JM109 (hMBC1HcDNA/pUC19), and FERM BP-5630 for *Escherichia coli* JM109 (hMBC1Lqλ/pUC19).

(5) Transfection into COS-7 cell

For the evaluation of the antigen-binding activity and the neutralizing activity of the hybrid antibodies and the humanized #23-57-137-1 antibodies, the above-prepared expression plasmids were expressed transiently in COS-7 cells. For the transient expression of the L-chain hybrid antibodies, each of the following combinations of plasmids were co-transfected into a COS-7 cell by electroporation using Gene Pulser (Bio Rad): hMBC1HcDNA/pCOS1 and h/mMBC1L(λ)/neo; hMBC1HcDNA/pCOS1 and m/hMBC1Laλ/neo; hMBC1HcDNA/pCOS1 and m/hMBC1Ldλ/neo; hMBC1HcDNA/pCOSI and hmmMBC1L(λ)/neo; and hMBC1HcDNA/pCOS1 and mhmMBC1L(λ)/neo. That is, a cell suspension (0.8 mL) of COS-7 cells in PBS(-) ($1\times10^7$ cells/mL) was added with each combination of the plasmid DNAs (10 μg each). The resultant solution was applied with pulses at an electrostatic capacity of 1,500V and 25 μF. After 10 min. of recovery period at room temperature, the electroporated cells were suspended in DMEM medium containing 2% Ultra Low IgG fetal calf serum (GIBCO), and then cultured using a 10-cm culture dish in a $CO_2$ incubator. After cultivating for 72 hours, a culture supernatant was collected and centrifuged to remove cell debris. The solutions thus prepared were provided for use in the ELISA below.

For the transient expression of the humanized #23-57-137-1 antibodies, the combination of plasmids of hMBC1HcDNA/pCOS1 and hMBC1Lxλ/pCOS1 (x = a-t) were co-transfected into a COS-7 cell using Gene Pulser (Bio Rad) in the same manner as described for the hybrid antibodies above. The culture supernatants were prepared and provided for use in the ELISA below.

The purification of the hybrid antibodies and the humanized antibodies from the COS-7 cell culture supernatants was performed using AffiGel Protein A MAPSII Kit (Bio Rad) in accordance with the instructions included in the kit.

(6) ELISA

(i) Determination of antibody concentration

An ELISA plate for determining antibody concentration was prepared as follows. Each well of a 96-well ELISA plate (Maxisorp, NUNC) was coated with 100 μl of a coating buffer (0.1 M $NaHCO_3$, 0.02% $NaN_3$) containing 1 μg/mL of goat anti-human IgG antibody (TAGO) and then blocked with 200 μl of a dilution buffer [50 mM Tris-HCl, 1 mM $MgCl_2$, 0.1 M NaCl, 0.05% Tween 20, 0.02% $NaN_3$, 1% bovine serum albumin (BSA); pH 7.2]. Each of the wells was added with each of the serial dilutions of the COS cell culture supernatant in which each of the hybrid antibodies and the humanized antibodies was expressed, or added with each of the serial dilutions of each of the hybrid antibodies and humanized antibodies in a purified form. The plate was incubated at room temperature for 1 hour and washed with PBS-Tween 20. Subsequently, each of the wells was added with 100 μl of alkaline phosphatase-conjugated goat anti-human IgG antibody (TAGO). The plate was incubated at room temperature for 1 hour and washed with PBS-Tween 20. Subsequently, each of the wells was added with 1 mg/mL of a substrate solution ("Sigma 104", p-nitrophenylphosphoric acid, SIGMA). The solution in each well was measured on its absorbance at 405 nm using Microplate Reader (Bio Rad) to determine the antibody concentration. In this determination, Hu IgG1λ Purified (The Binding Site) was used as the standard.

(ii) Determination of antigen-binding ability

An ELISA plate for determining antigen-binding ability was prepared as follows. Each well of a 96-well ELISA plate (Maxisorp, NUNC) was coated with 100 μl of a coating buffer containing 1 μg/mL of human PTHrP (1-34) and then blocked with 200 μl of a dilution buffer. Subsequently, each well was added with each of the serial dilutions of the COS-7 cell culture supernatant in which each of the hybrid antibodies and humanized antibodies was expressed, or added with each of the serial dilutions of each of the hybrid antibodies and humanized antibodies in a purified form. The plate was incubated at room temperature and washed with PBS-Tween 20. Subsequently, each well was added with 100 μl of alkaline phosphatase-conjugated goat anti-human IgG antibody (TAGO). The plate was incubated at room temperature and washed with PBS-Tween 20. Subsequently, each well was added with 1 mg/mL of a substrate solution ("Sigma 104", p-nitrophenylphosphoric acid, SIGMA). The solution was measured on its absorbance at 405 nm using Microplate Reader (Bio Rad).

(7) Confirmation of activities

(i) Evaluation of humanized H-chain

It was found that an antibody having a humanized H-chain version "a" and a chimeric L-chain exhibited the same level of PTHrP-binding activity as that of a chimeric antibody. This result suggests that the version "a" achieves the humanization of the H-chain V-region in the degree enough to evaluate the humanization. Therefore, the humanized H-chain version "a" was provided for use as a humanized antibody H-chain in the following experiments.

(ii) Activity of hybrid antibodies

(ii-a) FR1,2/FR3,4 hybrid antibody

When the L-chain was h/mMBC1L($\lambda$), no antigen-binding activity was observed. In contrast, when the L-chain was either m/hMBC1La$\lambda$ or m/hMBC1Ld$\lambda$, the same level of antigen-binding activity as that of the chimeric #23-57-137-1 antibody was observed. These results suggest that there is no problem with respect to FR3 and FR4 but there exist amino acid residue(s) that need to be replaced in FR1 and FR2 for the preparation of a humanized antibody.

(ii-b) FR1/FR2 hybrid antibody

When the L-chain was mhmMBC1L ($\lambda$), no antigen-binding activity was observed. In contrast, when the L-chain was hmmMBC1L($\lambda$), the same level of antigen-binding activity as that of the chimeric #23-57-137-1 antibody was observed. These results suggest that there is no problem with respect to FR1 but there exist amino acid residue(s) that need to be replaced in FR2 for the preparation of a humanized antibody.

(iii) Activity of humanized antibodies

Humanized antibodies each having the L-chain versions "a" to "t", were determined on the antigen-binding activity. As a result, it was found that the humanized antibodies having the L-chain versions "j", "l", "m", "o", "q", "r", "s" and "t" exhibited the same level of PTHrP-binding activity as that of the chimeric antibody.

(8) Establishment of CHO cell line capable of stable production of antibody

For establishing a cell line capable of stable production of humanized antibodies, each of the above-prepared expression plasmids was introduced into a CHO cell (DXB11).
That is, the establishment of a cell line capable of stable production of a humanized antibody was performed using each of the following combinations of plasmids as expression vectors for a CHO cell: hMBC1HcDNA/pCHO1 and hMBC1Lm$\lambda$/pCOS1; hMBC1HcDNA/pCHO1 and hMBC1Lq$\lambda$/pCOS1; and hMBC1HcDNA/pCHO1 and hMBC1Lr$\lambda$/pCOS1. The plasmids were co-transfected into a CHO cell by electroporation using Gene Pulser (Bio Rad). Subsequently, the expression vectors were separately cleaved with restriction enzyme PvuI to give linear DNA fragments. The resultant DNA fragments were extracted with phenol and chloroform and then precipitated with ethanol. The DNA fragments thus prepared were used in the subsequent electroporation. That is, the plasmid DNA fragments (10 $\mu$g each) were added to 0.8 mL of a cell suspension of CHO cells in PBS(-) ($1 \times 10^7$ cells/mL). The resultant solution was applied with pulses at an electrostatic capacity of 1,500V and 25 $\mu$F. After 10 min. of recovery period at room temperature, the cells thus treated were suspended in MEM-$\alpha$ medium (GIBCO) containing 10% fetal calf serum (GIBCO), and then cultured in a $CO_2$ incubator using 96-well plates (Falcon). On the day following the cultivation being started, the medium was replaced by ribonucleoside- or deoxyribonucleoside-free MEM-$\alpha$ selective medium containing 10% fetal calf serum (GIBCO) and 500 mg/mL of GENETICIN (G418Sulfate; GIBCO). From the culture medium, cells into which the antibody gene was introduced were selected. The culture medium was replaced by a fresh one. About two weeks after the medium replacement, the cells were observed microscopically. When a favorable cell growth was observed, the cells were determined on the amount of the produced antibodies by conventional ELISA for determination of antibody concentration as mentioned above. Among the cells, those which produced a larger amount of antibodies were screened.
The culture of the established cell line capable of stable production of antibodies was scaled up in a roller bottle using a ribonucleoside- or deoxyribonucleoside-free MEM-$\alpha$ medium containing 2% Ultra Low IgG fetal calf serum. On day 3 and day 4 of the cultivation, the culture supernatant was collected and filtered using a 0.2-$\mu$m filter (Millipore) to remove cell debris therefrom. The purification of the humanized antibodies from the culture supernatant of the CHO cells was performed using POROS Protein A Column (PerSeptive Biosystems) on ConSep LC100 (Millipore) in accordance with the appended instructions. The humanized antibodies were provided for use in the determination of neutralizing activity and examination of pharmacological efficacy on hypercalcemic model animals. The concentration and the antigen-binding activity of the purified humanized antibodies were determined by the ELISA system as mentioned above.

[REFERENCE EXAMPLE 5] Determination of neutralizing activity

The determination of neutralizing activity of the mouse antibodies, the chimeric antibodies and the humanized antibodies was performed using rat myeloma cell line ROS17/2.8-5 cells. The ROS17/2.8-5 cells were cultured in Ham'S F-12 medium (GIBCO) containing 10% fetal calf serum (GIBCO) in a $CO_2$ incubator. The ROS17/2.8-5 cells were seeded in each well of a 96-well plate at $10^4$ cells/100 µl/well and cultured for one day. After the cultivation was completed, the culture medium was replaced by Ham'S F-12 medium (GIBCO) containing 4 mM Hydrocortisone and 10% fetal calf serum. After cultivating for three to four days, the cultured cells were washed with 260 µl of Ham'S F-12 medium (GIBCO), and then added with 80 µl of Ham's F-12 medium containing 1 mM isobutyl-1-methyl xanthine (IBMX, SIGMA), 10% fetal calf serum and 10 mM HEPES. The resultant mixture was incubated at 37°C for 30 min.
The culture mediums of the mouse antibodies, the chimeric antibodies and the humanized antibodies to be tested for neutralizing activity were previously diluted serially in the following groups: [10 µg/mL, 3.3 µg/mL, 1.1 µg/mL and 0.37 µg/mL], [10 µg/mL, 2 µg/mL, 0.5 µg/mL and 0.01 µg/mL] and [10 µg/mL, 5 µg/mL, 1.25 µg/mL], 0.63 µg/mL and 0.31 µg/mL]. Each of the diluted antibody sample solutions was mixed with an equivalent amount of 4 ng/mL of PTHrP (1-34). The resultant mixed solution (80 µl) was added to each well. In each well, the final concentration of each antibody became a quarter of the above-mentioned concentration of the antibody, and accordingly the concentration of PTHrP (1-34) became 1 ng/mL. After the treatment at room temperature for 10 min., the culture supernatant was removed and the residue was washed with PBS three times. Subsequently, cAMP in the cells was extracted with 100 µl of a 0.3% HCl-95% ethanol and then evaporated using a water jet aspirator to remove the HCl-ethanol. The residue was dissolved in 120 µl of EIA buffer appended to cAMP EIA Kit (CAYMAN CHEMICAL'S) to extract the cAMP therefrom. The cAMP was determined using cAMP EIA Kit (CAYMAN CHEMICAL'S) in accordance with the instructions included in the kit. As a result, it was found that, among the humanized antibodies having the same level of antigen-binding activity as that of the chimeric antibody, those having L-chain versions "q", "r", "s" and "t" (in which the 91-position tyrosine was replaced by isoleucine) exhibited the closest neutralizing activity to that of the chimeric antibody, and those having a L-chain version "q" exhibited the strongest neutralizing activity.
The present specification encompasses the contents described in the specification and the drawings of the Japanese Patent Application No. 10-180143, which is a priority document of the present application.
All publications, patents and patent applications cited herein are incorporated herein by reference in their entirety.

INDUSTRIAL APPLICABILITY

The present invention provides a therapeutic agent for hypercalcemic crisis associated with malignant tumor comprising, as an active ingredient, a substance capable of inhibiting the binding between PTHrP and a receptor thereof.
The substance has more rapid onset and a longer duration of action compared with a control agent-administered group and a solvent-administered group (i.e., a control group). Accordingly, the substance is useful as a therapeutic agent for hypercalcemic crisis associated with malignant tumor for which emergency treatment must be considered.

Free Text for Sequence Listing:

SEQ ID NO: 1:    Synthetic DNA
SEQ ID NO: 2:    Synthetic DNA
SEQ ID NO: 3:    Synthetic DNA
SEQ ID NO: 4:    Synthetic DNA
SEQ ID NO: 5:    Synthetic DNA
SEQ ID NO: 6:    Synthetic DNA
SEQ ID NO: 7:    Synthetic DNA
SEQ ID NO: 8:    Synthetic DNA
SEQ ID NO: 9:    Synthetic DNA

SEQ ID NO: 10:   Synthetic DNA
SEQ ID NO: 11:   Synthetic DNA
SEQ ID NO: 12:   Synthetic DNA
SEQ ID NO: 13:   Synthetic DNA
SEQ ID NO: 14:   Synthetic DNA
SEQ ID NO: 15:   Synthetic DNA
SEQ ID NO: 16:   Synthetic DNA
SEQ ID NO: 17:   Synthetic DNA
SEQ ID NO: 18:   Synthetic DNA
SEQ ID NO: 19:   Synthetic DNA
SEQ ID NO: 20:   Synthetic DNA
SEQ ID NO: 21:   Synthetic DNA
SEQ ID NO: 22:   Synthetic DNA
SEQ ID NO: 23:   Synthetic DNA
SEQ ID NO: 24:   Synthetic DNA
SEQ ID NO: 25:   Synthetic DNA
SEQ ID NO: 26:   Synthetic DNA
SEQ ID NO: 27:   Synthetic DNA
SEQ ID NO: 28:   Synthetic DNA
SEQ ID NO: 29:   Synthetic DNA
SEQ ID NO: 30:   Synthetic DNA
SEQ ID NO: 31:   Synthetic DNA
SEQ ID NO: 32:   Synthetic DNA
SEQ ID NO: 33:   Synthetic DNA
SEQ ID NO: 34:   Synthetic DNA
SEQ ID NO: 35:   Synthetic DNA
SEQ ID NO: 36:   Synthetic DNA
SEQ ID NO: 37:   Synthetic DNA
SEQ ID NO: 38:   Synthetic DNA
SEQ ID NO: 39:   Synthetic DNA
SEQ ID NO: 40:   Synthetic DNA
SEQ ID NO: 41:   Synthetic DNA
SEQ ID NO: 42:   Synthetic DNA
SEQ ID NO: 43:   Synthetic DNA
SEQ ID NO: 44:   Synthetic DNA

**Claims**

1.  A therapeutic agent for hypercalcemic crisis comprising, as an active ingredient, a substance capable of inhibiting the binding between parathyroid hormone related protein (PTHrP) and a receptor thereof.

2.  The therapeutic agent for hypercalcemic crisis according to claim 1, wherein the substance is an antagonist against a PTHrP recepter.

3.  The therapeutic agent for hypercalcemic crisis according to claim 1, wherein the substance is an anti-PTHrP antibody.

4.  The therapeutic agent for hypercalcemic crisis according to claim 1, wherein the substance is a fragment of an anti-PTHrP antibody and/or a modified form of the fragment.

5.  The therapeutic agent for hypercalcemic crisis according to claim 3 or 4, wherein the antibody is a humanized or chimeric antibody.

6.  The therapeutic agent for hypercalcemic crisis according to claim 5, wherein the humanized antibody is humanized #23-57-137-1 antibody.

7.  The therapeutic agent for hypercalcemic crisis according to claim 3 or 4, wherein the antibody is of monoclonal type.

8. The therapeutic agent for hypercalcemic crisis according to any one of claims 1 to 7, wherein the hypercalcemic crisis is hypercalcemic crisis associated with malignant tumor.

# FIG. 1

# FIG. 2

# FIG. 3

# FIG. 4

# FIG. 5

# FIG. 6

## INTERNATIONAL SEARCH REPORT

| International application No. |
|---|
| PCT/JP99/03433 |

**A. CLASSIFICATION OF SUBJECT MATTER**
Int.Cl⁶ A61K45/00, 39/395 // C07K16/18, 16/46, C12N15/62, 5/16, C12P21/08

According to International Patent Classification (IPC) or to both national classification and IPC

**B. FIELDS SEARCHED**

Minimum documentation searched (classification system followed by classification symbols)
Int.Cl⁶ A61K45/00, 39/395 // C07K16/18, 16/46, C12N15/62, 5/16, C12P21/08

Documentation searched other than minimum documentation to the extent that such documents are included in the fields searched

Electronic data base consulted during the international search (name of data base and, where practicable, search terms used)
CAPLUS (STN), DDBJ

**C. DOCUMENTS CONSIDERED TO BE RELEVANT**

| Category* | Citation of document, with indication, where appropriate, of the relevant passages | Relevant to claim No. |
|---|---|---|
| X | Rosen, H. et al., The Effect of PTH Antagonist BIM-44002 on Serum Calcium and PHT Levels in Hypercalcemic Hyperparathyroid Patients, Calcif. Tissue Int. 1997, Vol. 61, No. 6, pp.455-459, Abstract, Discussion | 1, 2, 8 |
| X | JP, 7-165790, A (Tonen Corp.), 27 June, 1995 (27. 06. 95), Claims ; Par. Nos. [0008], [0009], [0037] (Family: none) | 1, 2, 8 |
| X | JP, 5-509098, A (The Regents of the University of California), 16 December, 1993 (16. 12. 93), Claims ; page 5, lower right column, line 16 to page 6, upper left column, line 2 ; page 8, lower left column, line 16 to lower right column, last line & WO, 92/00753, A1 & EP, 539491, A1 | 1, 2, 8 |

| ☒ Further documents are listed in the continuation of Box C. | ☐ See patent family annex. |
|---|---|

| * Special categories of cited documents: | "T" later document published after the international filing date or priority |
|---|---|
| "A" document defining the general state of the art which is not considered to be of particular relevance | date and not in conflict with the application but cited to understand the principle or theory underlying the invention |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 6 September, 1999 (06. 09. 99) | 14 September, 1999 (14. 09. 99) |

| Name and mailing address of the ISA/ Japanese Patent Office | Authorized officer |
|---|---|
| Facsimile No. | Telephone No. |

Form PCT/ISA/210 (second sheet) (July 1992)

EP 1 090 643 A1

# INTERNATIONAL SEARCH REPORT

International application No.

PCT/JP99/03433

C (Continuation). DOCUMENTS CONSIDERED TO BE RELEVANT

| Category* | Citation of document, with indication, where appropriate, of the relevant passages | Relevant to claim No. |
|---|---|---|
| X | JP, 4-228089, A (Kaneka Corp.), 18 August, 1992 (18. 08. 92), Abstract ; Claims ; Par. Nos. [0010] to [0020] (Family: none) | 1, 3-8 |
| X | WO, 98/13388, A1 (Chugai Pharmaceutical Co., Ltd.), 2 April, 1998 (02. 04. 98), Page 10, lines 4 to 13, page 80, line 9 to page 83, line 16 ; page 86, Example 9 to page 95, last line (Family: none) | 1, 3-8 |
| Y | OLSTAD, O.K et al., "Expression and Characterization of a Reconbinant Human Parathyroid Hormone Partial Agonist With Antagonisit Properties:Gly-hPTH(-1→+84), Peptides, 1995, Vol. 16, No. 6, pp.1031-1037, Reference as a whole | 1, 2, 8 |
| Y | Roubini, Eliahu et al., Synthesis of Fully Active Biotinylated Analogues of Parathyroid Hormone and Parathyroid Hormane-Related Protein as Tools for the Characterization of Parathyloid Hormone Receptors., Biochemistry, 1992, Vol. 31, pp.4026-4033, ABSTRACT, Page 4032, right column, lines 43 to 56 | 1, 2, 8 |

Form PCT/ISA/210 (continuation of second sheet) (July 1992)

39